# EUROPEAN PATENT APPLICATION

(11) **EP 2 502 852 A1**
(43) Date of publication of application: **26.09.2012**
(21) Application number: 11159808.2
(22) Date of filing: 25.03.2011
(51) Int. Cl.: B65D 83/04

(54) **Dispensing device**

(71) Applicant: DSM IP Assets B.V., 6411 TE Heerlen (NL)
(72) Inventor: The designation of the inventor has not yet been filed
(74) Representative: Viering, Jentschura & Partner

(57) **Abstract**

To provide a solution for proper dispensing a single unit dose of a solid substance when required, a dispensing device (3), especially a medical dispensing device, comprising an inner ring (8) provided with a receiving chamber (23) having a receiving opening (22) and a discharge opening (27), especially formed in a circular side wall (59) of the inner ring (8), and an outer ring (7) having a dispensing opening (34), especially formed in a side wall (65) of the outer ring (7), wherein the inner ring (8) and the outer ring (7) are rotatable arranged at a screw base element (6) having an opening (24) for delivering a substance therethrough, which substance is contained in a reservoir (2) which is detachably attachable to the dispensing device (3) to form a dispenser (1), is characterized in that in a first condition of arrangement the inner ring (8) and the outer ring (7) are engaged to each other for commonly performing a first rotational movement relative to the screw element (6), which first rotational movement comprises a first step (I) of commonly releasing the inner ring (8) and the outer ring (7) from a common starting position where said inner ring (8) is in a locked position with the screw base element (6) and a second step (II) of commonly rotating the inner ring (8) and the outer ring (7) along a first rotational direction (21) into a first stop position, where the receiving opening (22) of the receiving chamber (23) comes into a receiving communication with the opening (24) provided in the screw base element (6) and a third step (III) of commonly rotating the inner ring (8) and the outer ring (7) back into the starting position of the second step (II) along a second rotational direction (28) opposite to the first rotational direction (21) and a fourth step (IV) of commonly bringing the inner ring (8) and the outer ring (7) back into their common starting position, and that in a second condition of arrangement the inner ring (8) and the screw base element (6) are engaged to each other for allowing the outer ring (7) to be rotated relatively in respect to the inner ring (8) and the screw base element (6) during a second rotational movement, which second rotational movement comprises a fifth step (V) of rotating the outer ring (7) further along the second rotational direction (28) starting from the starting position into a second stop position, where the discharge opening (27) of the receiving chamber (23) is in dispensing communication with the dispensing opening (34) of the outer ring (7) and a sixth step (VI) of rotating the outer ring (7) along the first rotational direction (21) back into the starting position.

## Description

The invention relates to a dispensing device, especially a medical dispensing device, comprising an inner ring provided with a receiving chamber having a receiving opening and a discharge opening, especially formed in a circular side wall of the inner ring, and an outer ring having a dispensing opening, especially formed in a side wall of the outer ring, wherein the inner ring and the outer ring are rotatable arranged at a screw base element having an opening for delivering a substance there through, which substance is contained in a reservoir, which is detachably attachable to the dispensing device to form a dispenser.

Such kinds of dispensing devices are used for dispensing solid preparations stored and contained in a reservoir container or bottle, which is permanently or which will be brought into a dispensing communication with the dispensing device. Solid preparations may be medical or pharmaceutical preparations, nutritional preparations, confectionary preparations and all other kinds of solid materials, which are of a solid configuration. Such preparations or substances may be pills, capsules, tablets, particles, pallets, agglomerations etc. One special field for application of this kind of solid preparations is the field of medication. Here solid pharmaceutical preparations are generally prescribed by a physician to the patient; however some pharmaceutical preparations can be obtained without prescription (so-called "over-the-counter medication") . Whether the medication is prescribed by a physician or obtained over-the-counter, best results are generally obtained when the medication is taken at regular intervals so that the effective ingredient in the medication is replenished at regular and precise intervals. Daily or otherwise regular usage is not only important in the normal use of medication so as to have as regular a dosage pattern as possible, but it is even more important in clinical trials. Before any drug is allowed to be prescribed by a physician, the drug must be registered at local agencies, such as the FDA (Food and Drug Administration) in the USA and the European Medicines Agency (EMEA) in Europe. Only after registration may the drugs be put on the market and prescribed and sold for pharmaceutical preparations. To pass the registration procedure, large amounts of data regarding the drug need to be collected and supplied to the authorities. One part of these data is the result of clinical trials. In several phases of the clinical trials, patients are administered a certain dose of the drug. The effects of the drug on the patient are carefully followed, so as to be able not only to determine the effect of the drug but also possible side effects. For the correct determination of the side effects, it is important that the patient strictly follows the prescribed medication regime, or in other words that he/she complies with the regime. When he/she deviates from the regime he/she is considered not to be compliant. The consequence of non-compliant behavior will strongly depend on the kind of drug he/she was taking. With some drugs, the effect may not be serious, while with others, it can lead, for example, to symptoms of poisoning. When a patient who is participating in a clinical trial shows these symptoms of poisoning, the conclusion could, possibly erroneously, be drawn that the symptoms of poisoning and the poisoning itself are adverse effects of the drug itself. Therefore it would be an advantage to the patient, researcher and physician participating in the clinical trials to be able to follow the patient's compliance behavior.

Another factor influencing a patient's compliance is posed by new developments in therapeutics, such as the use of cocktails of pharmaceuticals and the use of chrono-therapeutics. Since the recent discovery that combinations of pharmaceuticals are more effective than mono-therapies in treating AIDS, some cancers and infectious diseases, some of these patients need to take considerable numbers of pills each day without missing doses, else resistance to treatment could develop. Chrono-therapeutics are used for illnesses that fluctuate with the biological rhythm of a patient such as for example asthma. Asthma is often more severe at night than during the day. Therefore it is not necessary to administer a steady dose. A preferred treatment should match the medicine levels to the symptoms. This is exactly what can be done with chrono- therapeutics. However it is essential that the patient is very precise in complying with the prescribed (time) scheme.

Patients sometimes tend to forget their medication. This forgetfulness is strongly dependent on external factors, such as for example holidays, traveling through different time-zones and stress. So patients that experience these situations more often are at a higher risk for non-compliance. Another group of patients with an increased risk for non-compliance are patients with a chronic disease. The chronic disease causes the character of the medication also to be chronic. As it is easier to obey prescription rules for only 10 days than for 10 years, especially patients with a chronic disease may have difficulties in complying with the medication regime as prescribed by their physician.

Again another group of patients with an increased risk of non-compliance are elderly people who suffer from forgetfulness.

All the factors mentioned above in addition to the recent developments can make it difficult for a patient to take his medication right in time; that is: to comply with the medication regime prescribed by the physician. This is reflected in the fact that less than 70% of prescribed medication is actually consumed. For chronic diseases, such as for example hypertension and diabetes this is even lower: approximately 50%. When diseases or conditions are treated sub-optimally, symptoms and complications may worsen, leading to increased use of hospital and emergency services, office visits and other medical resources. Increased adherence may generate medical savings that more than offset the associated increases in drugs costs. In addition to the costs, medication that is prescribed but not consumed must be disposed of in a sensible way, giving rise to additional costs. The non-consumed medication can pose a health risk when it is not brought back to the pharmacy or is not disposed of in another sensible way. For example a child can get hold of it and think it is candy and eat it with all the health risks going with it. Or the presence of the medication in some system can generate immunity against the active ingredient in the medication.

For the reasons described above, either separately or combined, it would be desirable to have means available to aid a patient, researcher, physician and/or pharmacist to increase the rate of compliance for taking medication and possibly not only increase the rate, but also increase the convenience, that is "the ease" with which compliance can be reached. In addition to the mentioned persons, it could also be advantageous for family and/or friends and/or other care takers around the patient.

Although the advantages are described above in connection to solid pharmaceutical preparations, the advantages can apply fully, or at least partially, to other fields where solid preparations are supplied to or taken by a person. An example of such another field outside the pharmaceutical field is the nutritional field, or the field of confectionary. The device according to the present invention can equally well be used in these and other fields; it is not limited to the pharmaceutical field. Therefore, the term "dispensing device" refers to a device in a very brought sense as well as the term "substance" and they cover all applications and application forms as mentioned before and hereafter.

The wording "substance" is meant to encompass each and any form of solid dosage and preparation of a certain predetermined amount and shape. With solid dosage is meant a dosage that during normal use by the user, such as for example a patient, retains its shape. It encompasses a dosage of a liquid or liquid-like substance that is captured within a solid skin. Examples of solid preparations are tablets, dragees, capsules and pills, wherein later on the term "pills" is used as a synonym for all these solid preparations mentioned before. Examples of solid preparations with a liquid or liquid-like substance captured in a solid skin are dragees with a gel-like substance contained in it. With solid pharmaceutical preparation is meant a solid preparation containing at least one pharmaceutically active compound. With solid nutritional preparation is meant a solid preparation containing at least one nutritionally active compound.

A dispensing device for medication is known from US2006/0283876. The dispenser according to US2006/0283876 comprises elements that can indicate the moment the medicament should be dispensed and it contains means to initiate an alarm at the moment that the medicament should be taken according to a pre-set frequency scheme. This dispenser can therefore help a patient to take the medicament at regular pre-set intervals according to a pre-set frequency.

A disadvantage of the dispensing device according to US2006/0283876 is that it is impossible to analyze the patient's compliance to the pre-set medication regime. Some kinds of medication need to be taken at very strict intervals. Not obeying this regime can cause severe harm to the patient. Therefore the availability of this kind of information would be very helpful to, for example emergency doctors when the patient, due to the non-observance, needs to be brought to the hospital.

A dispensing device, especially for dispensing solid preparations, comprising a housing having a dispensing orifice, and a collar member having a receiving opening, and a passage member having a receiving chamber, is known from the US 2,962,190 A. This document discloses a dispensing device wherein a receiving chamber comprising a receiving opening and a dispensing opening is movably arranged within a housing, wherein in a first position the receiving opening of the receiving chamber is in a receiving communication with a housing providing the solid preparations and wherein the receiving chamber is in a second position providing a dispensing communication between its dispensing opening and a dispensing orifice of the housing. It is a disadvantage of this known dispensing device that the compartment of the receiving chamber receiving the solid substances or preparations which shall be dispensed may receive more than one of these solid preparations. Thus, it is difficult or nearly impossible to assure that only one single dose will be dispensed.

A dispensing device comprising an electronic circuit and/or an energy supply for monitoring, controlling and/or communication as well as a method of monitoring a patient's medication compliance using a dispenser is known from WO 2007/081947 A. These known device and method comprise the use of a relatively complex constructed dispenser which might be improved under convenience aspects.

A method of monitoring a patient's medication compliance involves utilizing an automatic compliance monitoring device, which tracks patient compliance data, along with a wave energy transmitter and power source, wherein a receiver is connected to a computer. The dispenser or the computer is programmed to calculate compliance requirements by for example the number of cap openings. Such a method is known from EP 0 526 166 A1. A disadvantage of the method described in EP 0 526 166 A1 is that the compliance determination is vulnerable to manipulation, either deliberately or accidentally. For example, when a patient opens his medication bottle to see how many pills are still in there and to determine whether he has to ask for a refill, the automatic compliance monitoring device records an opening of the bottle and records, falsely, compliance.

Solid pharmaceutical preparations and/or solid nutritional preparations and/or solid confectionary preparations pose certain requirements on the atmosphere wherein they are contained. When the quality of the atmosphere surrounding the solid pharmaceutical preparations and/or solid nutritional preparations and/or solid confectionary preparations is not sufficient a reasonable shelf life can't be obtained. With a reasonable shelf life is meant a period of usually between six months and three years, typically at least two years. One factor influencing the quality of the atmosphere within the container is the relative humidity. Relative humidity (RH) has here and hereinafter its usual meaning, being the ratio of the actual humidity over the saturated humidity at the same temperature. To reach the reasonable shelf life indicated above, the relative humidity inside the container has generally to be maintained below 50%, preferably less than 40%, more preferably less than 30%.

A container for holding solid pharmaceutical preparations is known from US2007/0163917. US2007/0163917 describes a package for pharmaceutical dosage forms, wherein the pharmaceuticals inside pose certain requirements on the level of oxygen and moisture present in the package. The pharmaceutical dosages are packed in a bottle. To decrease or at least maintain the oxygen level in the bottle at an acceptable level, a self-activated metal-based oxygen absorber is placed in one sub-container. To decrease or at least maintain the moisture level in the bottle at an acceptable level, a desiccant is placed in another, second, sub-container. The sub-container with the desiccant is covered on both open ends with membranes so as to allow the air in the container to enter the desiccant sub-container. The sub-containers can be separate units or unitary, that means they are fabricated together as separate compartments within a single unit.

A disadvantage of the construction chosen in US2007/0163917 is that the production process is difficult as first a bottle must be constructed, wherein at least two separate sub-containers must be placed. Additionally, the filling of the bottle with the pharmaceutical dosages is difficult as the bottle already contains the sub-containers.

From all the above it can be taken, that there is a need for a dispensing device, especially one which is able to dose only a single dose even when the dispensing device or a reservoir attached thereto contains a plurality of doses. There is a further need that such a dispensing device can additionally capture, record and transmit the dispensing event to an external device. Such a device is a combination of a dispenser with electronics, communication software and a database management system. These devices aim at improving the compliance of patients to their medication prescription and the medication that patients take every day is measured by electronics in the dispenser. Such a unit dose dispenser can be used for:
- Clinical Trials, wherein the unit dose dispenser can be used to more accurately record information about patient compliance in taking medication. The compliance data can also be integrated with other diagnostic testing to provide integrated data on each patient for the clinical trial. Such a dispenser can be used by companies that manage clinical trials to accurately collect data with less labor.
- Prescriptions for Maintenance Drugs, wherein the unit dose dispenser can be used by a pharmacist as a compliance tool. After a patient takes the prescription, the dispenser is returned to the pharmacy where data is downloaded to a database. The pharmacist can evaluate the compliance and coach behavior on how to take the prescription (if necessary). The pharmacist can also share data from the unit dose dispenser with the prescribing physician.
- Pharmaceutical, wherein the unit dose dispenser can be used with specialized generics or used to extend patent protection on branded drugs.
- Hospital, Nursing Home, Assisted Living, wherein the dispenser may contribute to improve compliance and more efficiency dispense medication.

It is an object of the present invention to overcome or at least diminish the above mentioned disadvantages and to provide a solution for proper dispensing a single unit dose of a solid substance when required.

It is another object of the invention to provide means for increasing the rate of patient's compliance. It is another object of the invention to control and/or monitor relative humidity and/or temperature in a dispensing system.

This object is achieved by a dispensing device according to claim 1.

Favourable and advantageous embodiments of the invention are part of the depending claims.

The invention provides a dispensing device and a dispenser, which allows the dispensing of a substance, especially a solid preparation, in a unit dose manner. Due to the rotational movement of the outer ring and the inner ring relative to a screw base element, which is torque proof attached to a reservoir containing the solid substances in a first condition of arrangement, and the rotational movement of the outer ring relative to the inner ring and the screw base element in a second condition of arrangement it is possible to design and form a delivery of a solid substance to a receiving chamber and to dispense a single solid dose of the substance. During the first condition of arrangement a sequence of four steps allows to singularize a solid substance or pill from a plurality of solid substances or pills contained in a reservoir or bottle and to deliver it to the receiving chamber provided within the dispensing device. During the second condition of arrangement, another sequence of two steps allows it to dispense the single dose of a solid substance from the receiving chamber out of the dispensing device. To be detachably detachable to a reservoir and to form a dispenser, the dispensing device is equipped with a bottle adapter.

For separating and singularizing a single dose of a solid substance from a plurality of substances or pills contained in the reservoir the invention further provides that the dispensing device comprises a funnel, which has a circular and inclined ramp, which is such positioned with respect to a neck of the reservoir and such designed and dimensioned with respect to the dimensions of the substance or pill that it allows to singularize a substance or pill from a plurality of substances or pills contained in the reservoir and to deliver it in a single dose manner to the opening provided in the screw base element.

Advantageously, the funnel is arranged at a position to be hold by the bottle adapter and the screw base element.

It is further possible to provide the dispensing device and the dispenser with electronic means for monitoring, controlling and data communication, which additionally enhances therapy compliance. Therefore, the dispensing device according to the invention is further characterized in that it comprises a printed circuit board, which is torque proof arranged within the inner ring to be movable together with the inner ring in rotational directions and relatively with respect to a stop member, which stop member is torque proof connected to the screw base element.

To provide the possibility of switching electronics provided on the printed circuit board (PCB), the invention further comprises a printed circuit board base, which is torque proof arranged within the inner ring at a position between the printed circuit board and the stop member and to be movable together with the inner ring and the printed circuit board in rotational directions and relatively with respect to a stop member.

To realize the switching mechanism, the invention further provides that the printed circuit board is provided with at least one contact element, which contact element, preferably on its surface facing the printed circuit board, interacts with at least one switch provided at the printed circuit board and which contact element, preferably on its surface facing a surface of the stop member, interacts with this surface of the stop member and/or a groove or cavity provided therein, to be switched during rotational movement of the inner ring in the first rotational direction and/or the second rotational direction.

A further advantage of the invention consists in that the dispensing device, especially the printed circuit board and/or the inner ring, is/are provided with electronics and/or at least one energy source and/or at least one detecting means detecting and/or monitoring the dispense of a substance or pill from the dispenser and/or at least one humidity sensor and/or at least one temperature sensor. Hereby, it may be of an advantage that the dispensing device comprises at least one means for electronically and/or mechanically identifying the kind of reservoir attached to the dispensing device, wherein a means for electronically identifying the kind of reservoir preferably comprises a barcodce reader.

According to another aspect of the invention, a dispensing device may comprise an antenna to which especially the printed circuit board and/or the electronics is/are connected to.

Further, the dispensing device is provided with at least one means for at least storing information relating to the reservoir and/or the dispense event of a substance or a pill and/or a temperature and/or a humidity in the reservoir and/or for at least transmitting information thereabout to an external device.

For communicating with a data system or other devices and to provide the dispensing device with performances and/or properties and/or quality characteristics of a mobile phone, it is according to a further aspect of the invention that the dispensing device comprises means allowing the device to communicate via Global System for Mobile Communications (GSM).

According to another main aspect of the invention the dispensing device is characterized in that it is a medical dispensing device and that it comprises means for communicating with a second medical device to coordinate the administration of medication from the dispensing device and the second dispensing device and/or to receive information from the second device.

When realizing such a communication system, it may be advantageous that the dispensing device comprises an electronic circuit provided with a transceiver, preferably a near field communication (NFC) transceiver - that wirelessly transmits the information associated with the dispensing event to an external device, which is another aspect of the invention.

According to another aspect of the invention it is still advantageous and advisable, if the dispensing device is part of a system comprising an external data base, which stores information received from and to be submitted to the dispensing device and to which the dispensing device is, preferably wirelessly, communicating and vice versa.

Finally, the invention is characterized in that the dispensing device tracks at least one of a patient's medicine compliance data.

The invention will now be described in reference to the accompanying drawings of particular embodiments which are given by way of non-limiting example. In the drawings shows:
- Fig. 1-6:: the dispenser according to the invention during different steps and stages of a dispensing cycle,
- Fig. 7:: the dispenser of Fig. 1-6 and according to the invention in an exploded view,
- Fig. 8a and 8b:: a perspective view of a bottle adapter, respectively,

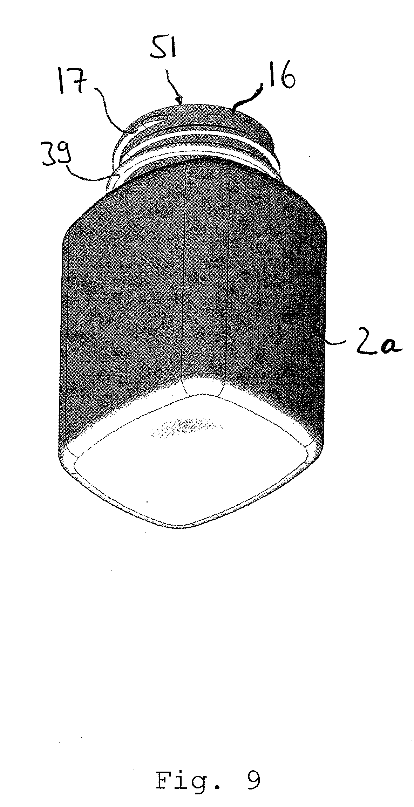

- Fig. 9:: a perspective view of a reservoir or bottle,

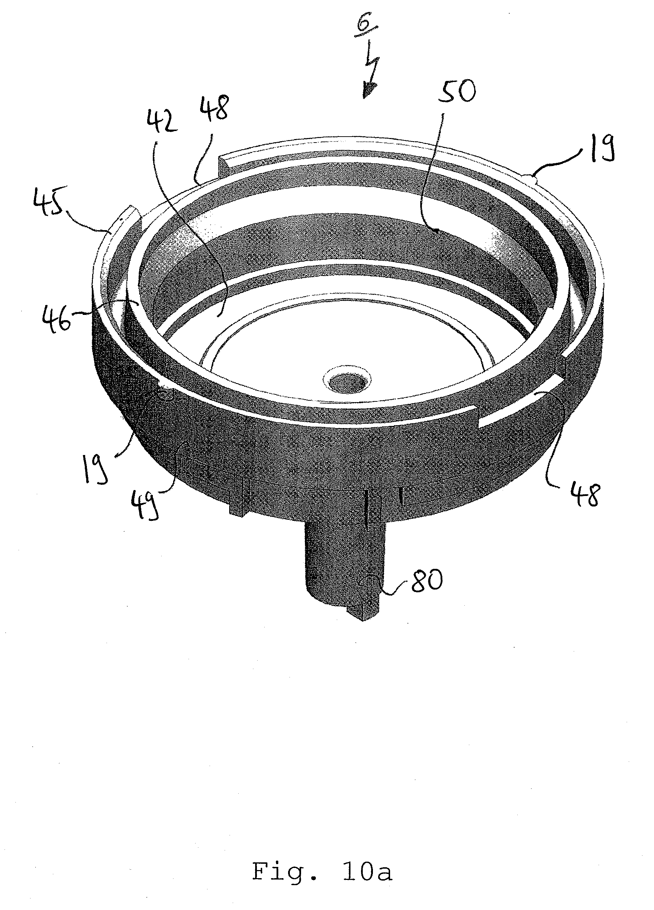

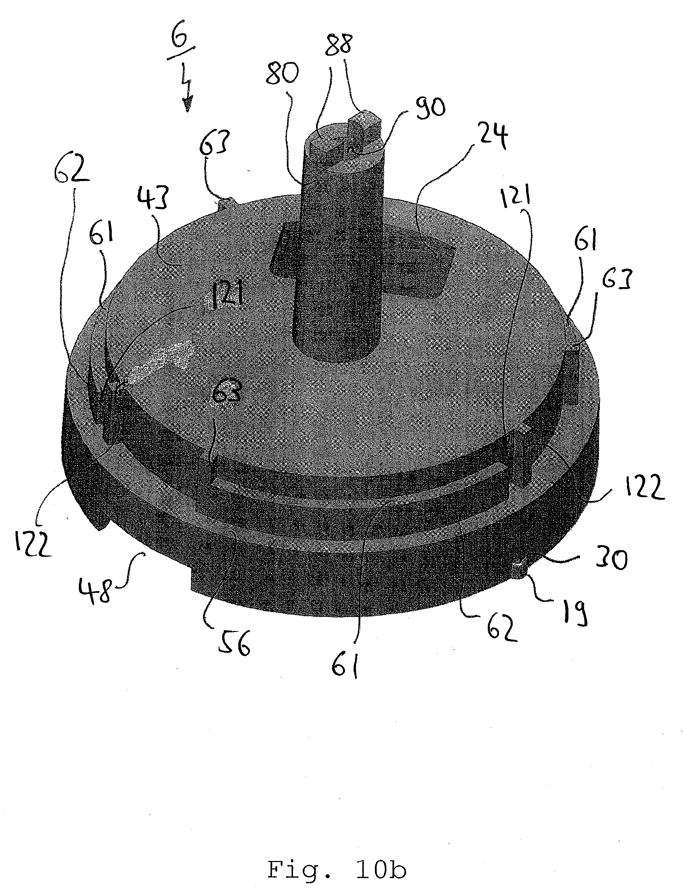

- Fig 10 and 10b:: a perspective view of a screw base element, respectively,

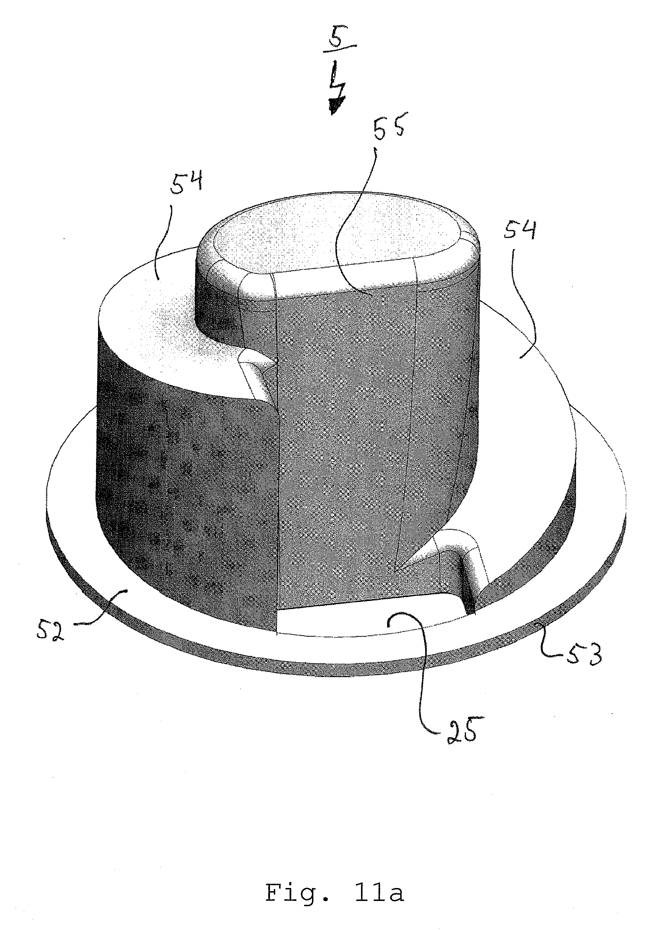

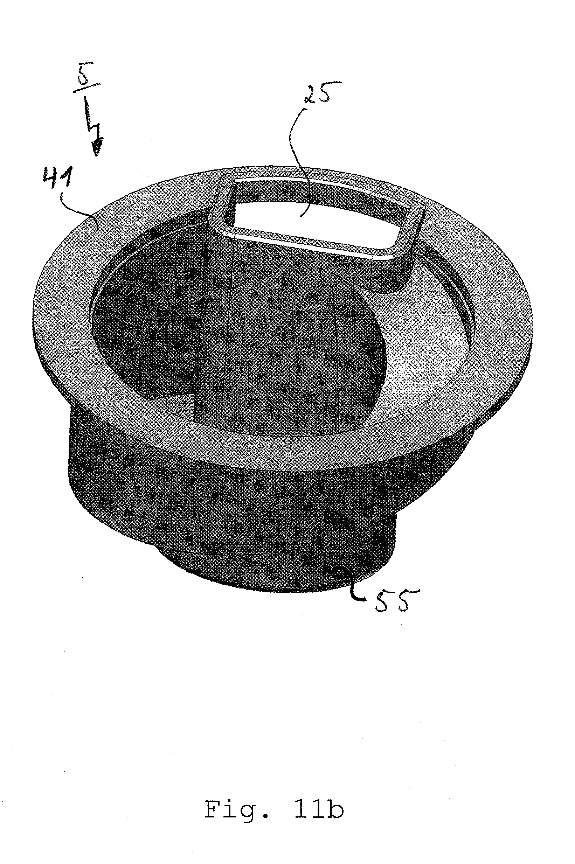

- Fig. 11a and 11b:: a perspective view of a funnel, respectively,

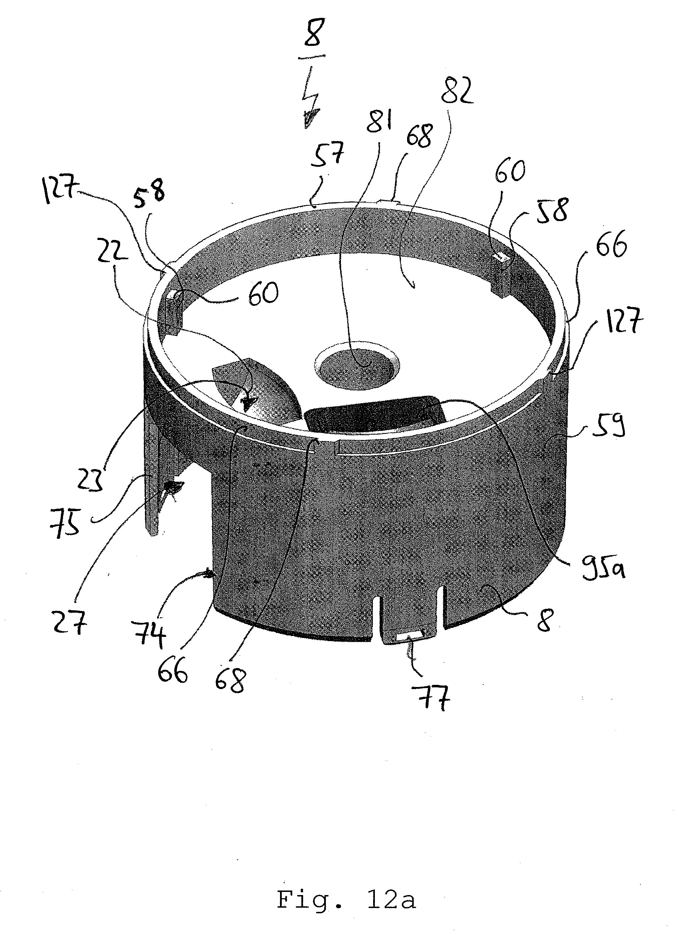

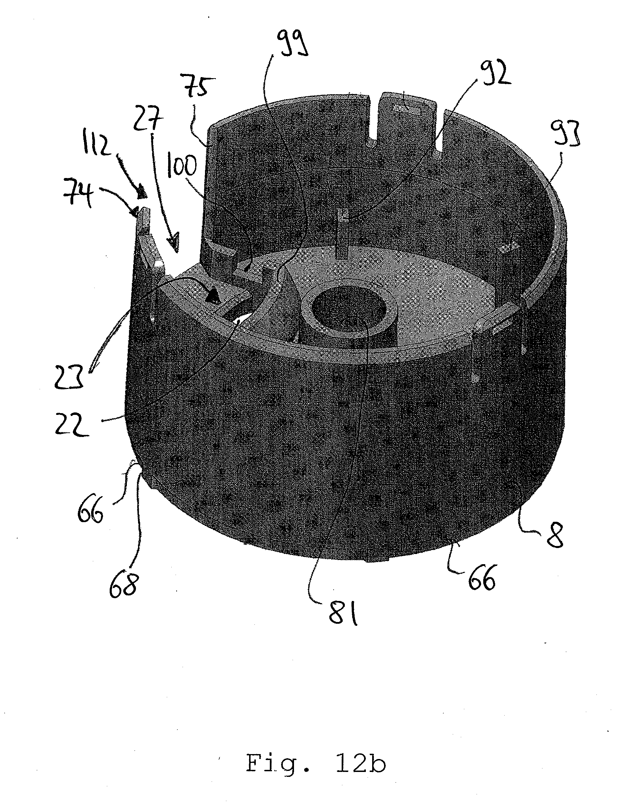

- Fig. 12a and 12b:: a perspective view of an inner ring, respectively,

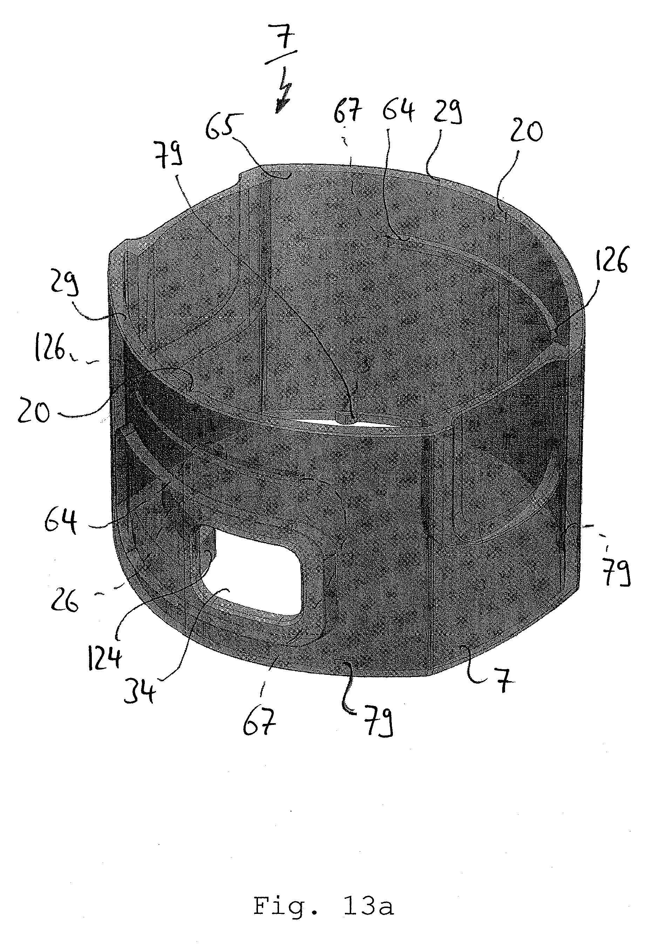

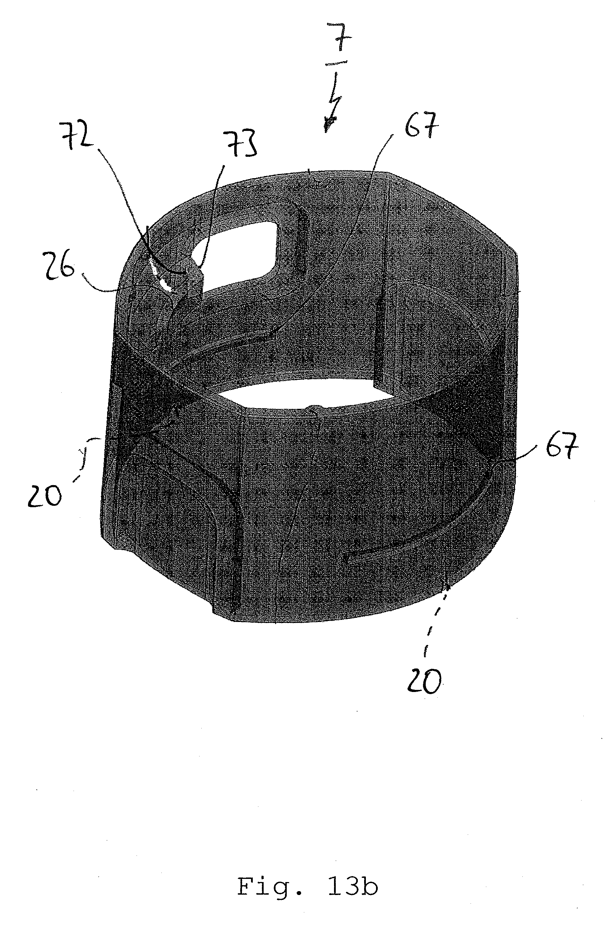

- Fig. 13a and 13b:: a perspective view of an outer ring, respectively

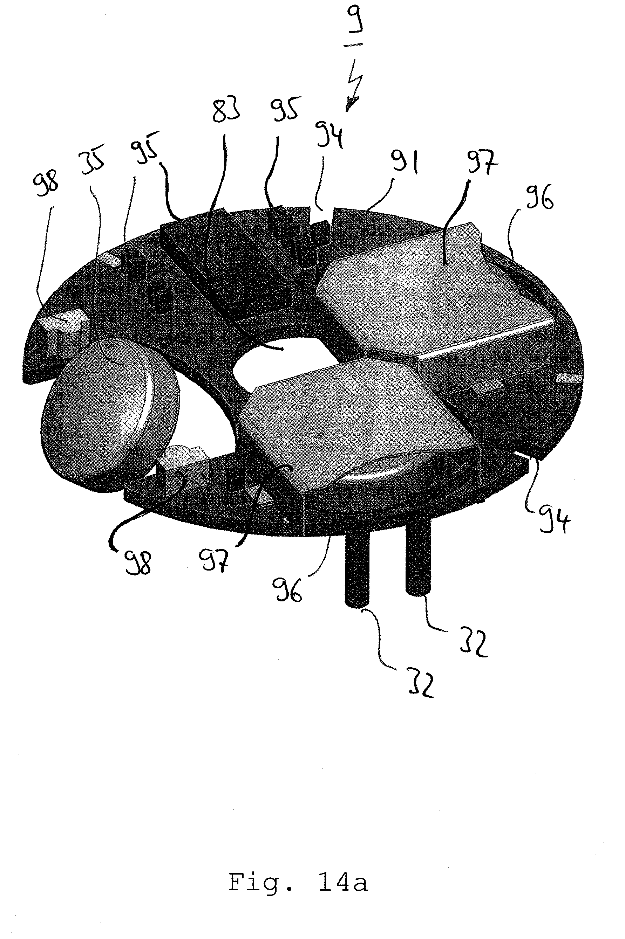

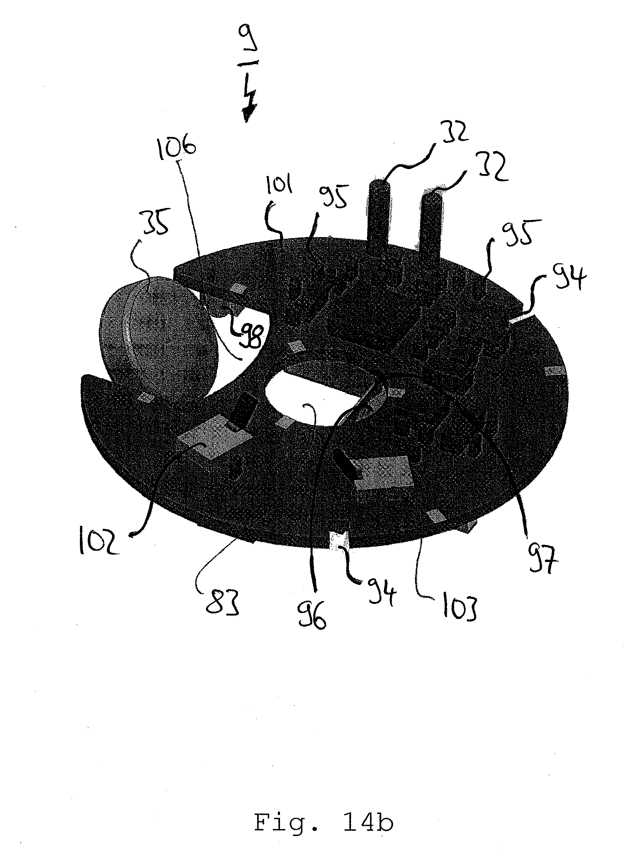

- Fig. 14a and 14b:: a perspective view of a printed circuit board, respectively

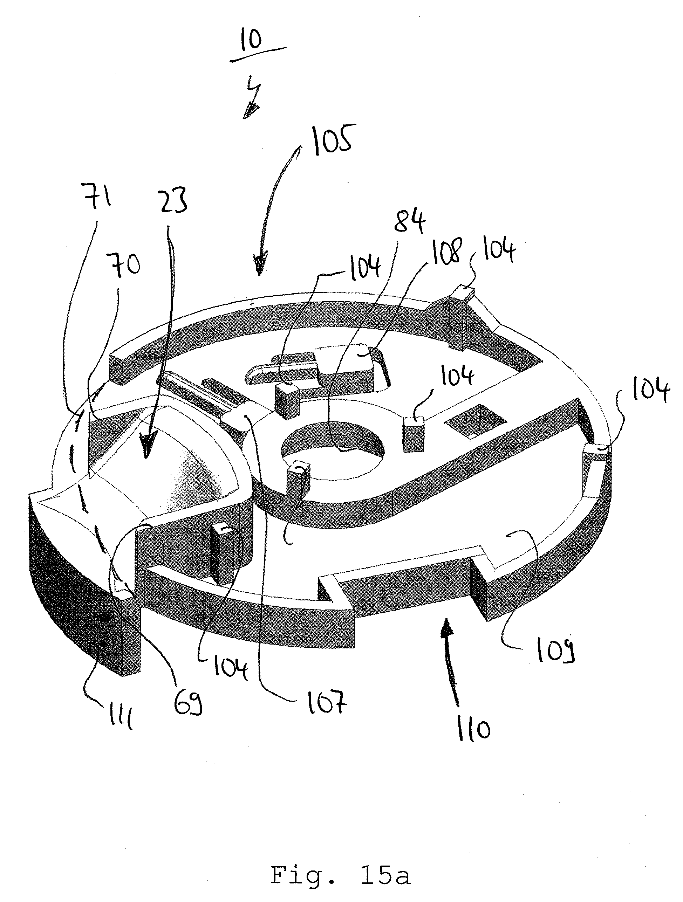

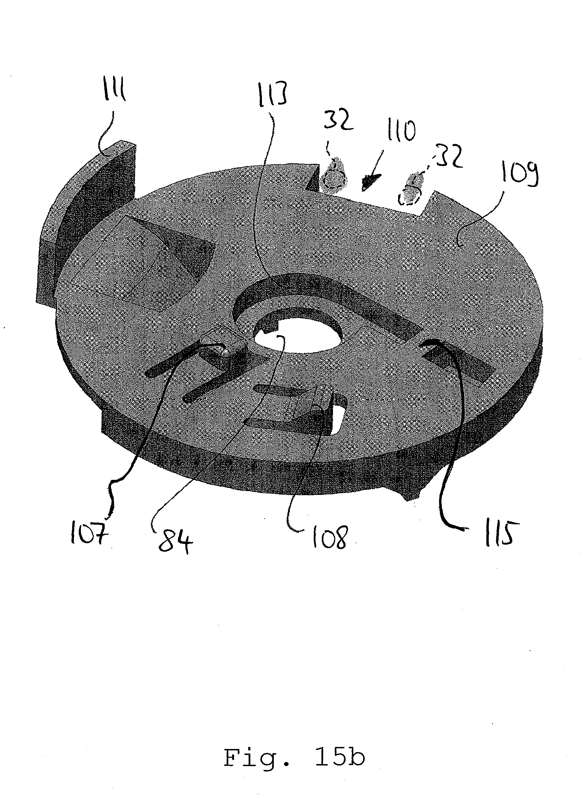

- Fig. 15a and 15b:: a perspective view of a printed circuit board base, respectively

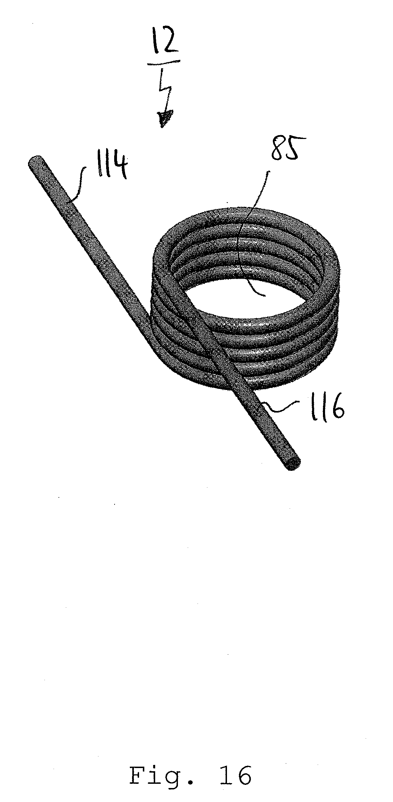

- Fig. 16:: a perspective view of a spring,
- Fig. 17a and 17b:: a perspective view of a stop member, respectively

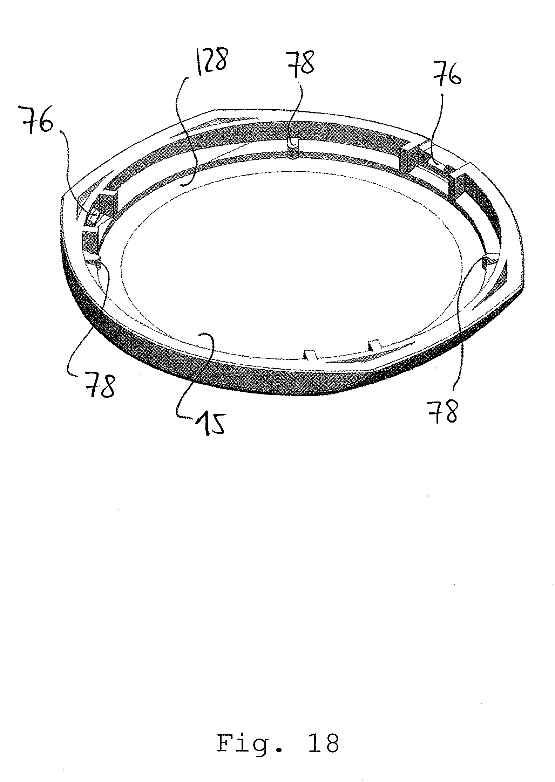

- Fig. 18:: a perspective view of a cap,
- Fig. 19:: a cross sectional view of a dispenser according to the invention,
- Fig. 20:: a diagrammatic illustration of a block diagram of an electronic circuit realized on a printed circuit board,
- Fig. 21:: a diagrammatic view of a system for communicating with a dispenser,
- Fig. 22:: a diagrammatic illustration of a block diagram of an electronic reader/writer device and
- Fig. 23:: a diagrammatic view of a therapy compliance monitoring and communication means.

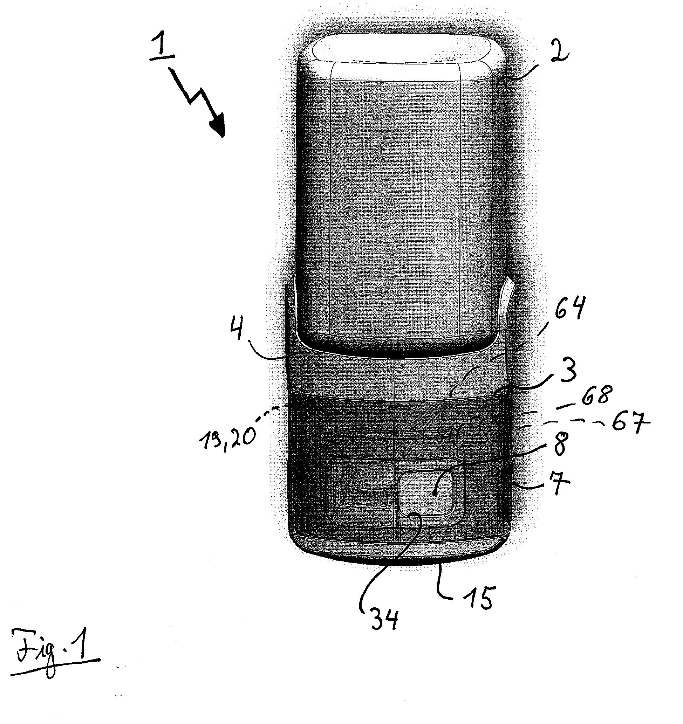

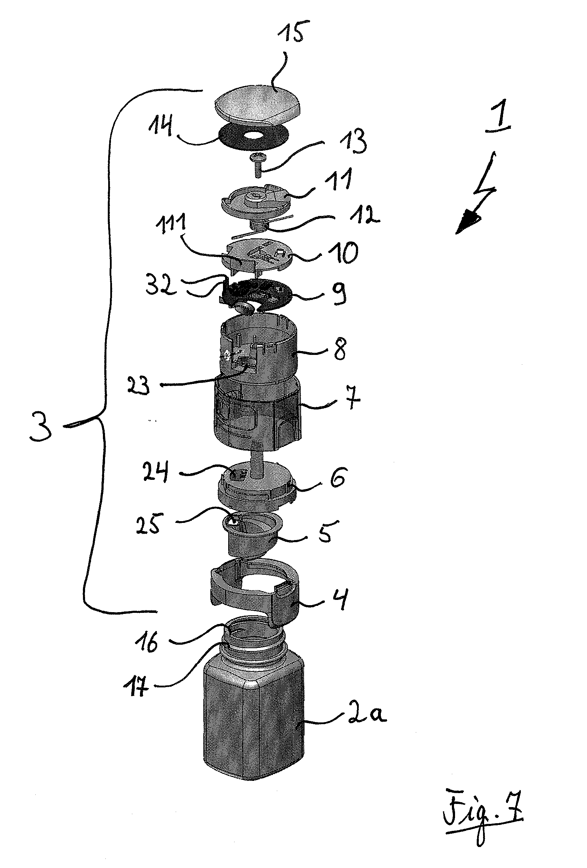

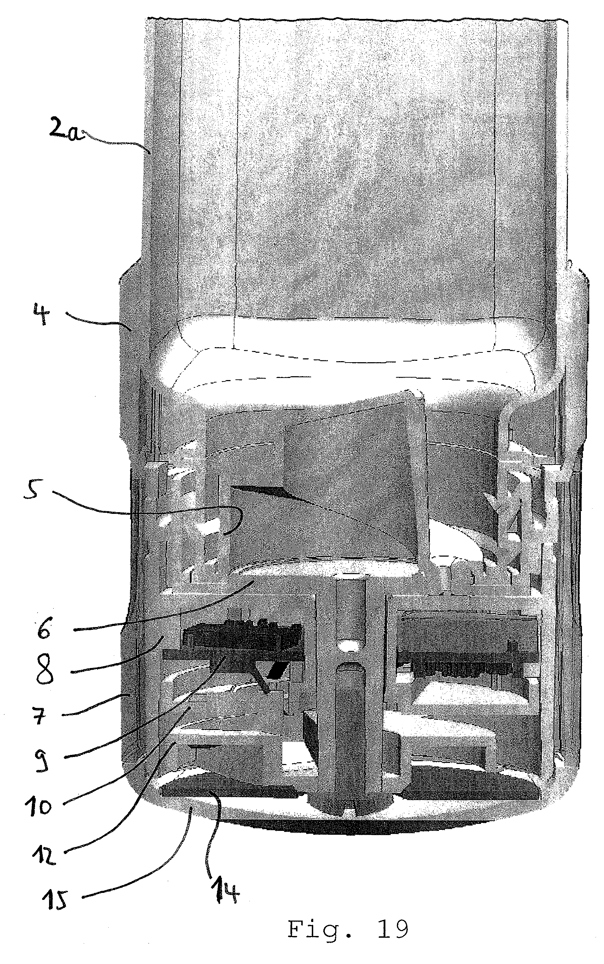

An embodiment of a dispensing device 3 according to the invention is shown in Fig. 1-7 and 19. According to this embodiment a dispenser 1 comprises a reservoir bottle 2 and 2a in the form of a dispensing device 3 attached to the bottle 2a. The bottle 2a comprises a neck 16 provided with a screw threat 17 on its outer surface and the dispensing device 3 comprises screw thread means provided in a screw base element 6 corresponding thereto so that it is possible to separate the bottle 2a and the dispensing device 3 by unscrewing. This makes it possible to exchange one bottle 2a against another one, for instance in case that a first bottle 2a has been emptied. Normally, the bottle 2a contains solid particles as pills, capsules or other pharmaceutical or nutritional or confectionary products or substances.

Although the bottle 2a and the dispensing device 3 are connected by a screw threat, other solutions for constituting a removable and detachable connection with respect to the bottle 2a and the dispensing device 3 maybe realized, for instance a bayonet coupling or another means selected from all kinds of qualified and well known coupling and/or fitting members and/or constructions.

The dispensing device 3 comprises a bottle adapter 4, a funnel 5, the screw base element 6, an outer ring 7, an inner ring 8, a printed circuit board (PCB) 9, a printed circuit board base 10, a stop member 11, a spring 12 connected to and positioned between the printed circuit board base 10 and the stop member 11, a screw 13 to be connected to the screw base element 6, an antenna 14 and a cap 15.

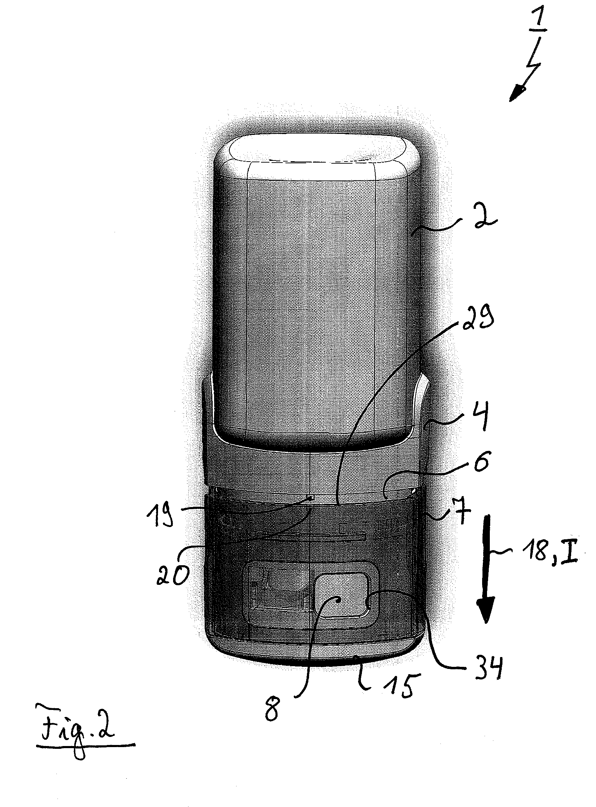

The dispenser 1, especially the dispensing device 3 is designed for and suitable for delivering substances in a unit dose manner, wherein the substances are contained in the bottle 2a. For dispensing such a unit dose the outer ring 7 has to be moved, for instance by pulling, relatively to the bottle adapter 4 in a longitudinal direction as indicated by arrow 18 in Fig. 2 in a first step I of a dispensing cycle. Such a longitudinal movement brings burlings 19, which are provided at the rim portion of the outer surface of the screw base element 6, out of an engagement with corresponding recesses 20 provided at the inner surface of the outer ring 7. By pulling in the direction of the arrow 18 the elements or members outer ring 7, inner ring 8, printed circuit board (PCB) 9, printed circuit board base (PCB base) 10 and the cap 15 are all moved together as a single unit, subsequently referred to as a first member entity, relative to another unit, subsequently referred to as a second member entity, constituted by the bottle adapter 4, the funnel 5, the screw base element 6, and the stop member 11 which is connected to the screw base element 6 by the screw 13. The bottle 2a is torque proofed connected to this second member entity. The relative movement of these two member entities with respect to each other when pulling the first member entity in the longitudinal direction 18 as indicated by arrows 18 in the first step I of a respective dispensing cycle causes a compression of the spring 12.

The first member entity constitutes a first condition of arrangement wherein the inner ring 8 and the outer ring 7 are engaged to each other for commonly performing a first rotational movement relative to the screw base element 6. The second member entity constitutes a second condition of arrangement wherein the inner ring 8 and the screw base element 6 are engaged to each other for allowing the outer ring 7 to be rotated relatively with respect to the inner ring 8 during a second rotational movement.

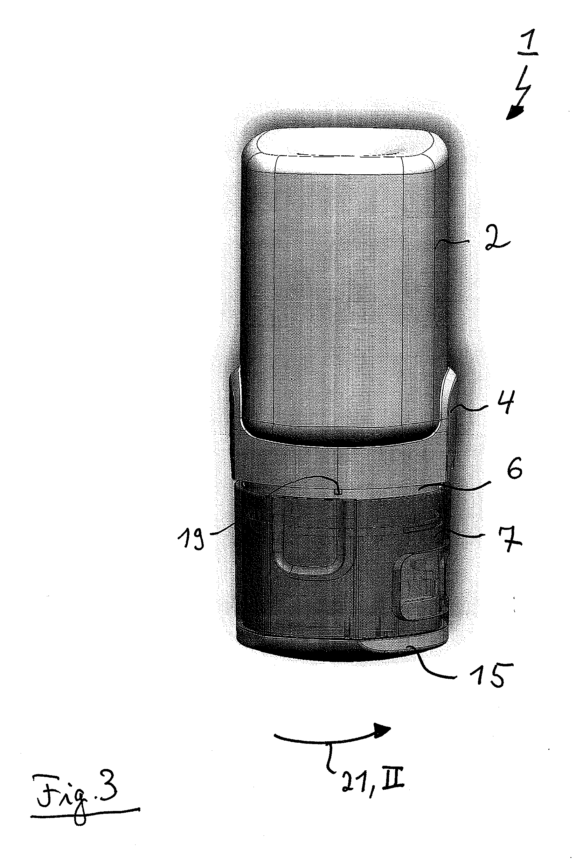

Subsequently, in a second step II of the respective dispensing cycle, the first member entity is rotated relatively to the second member entity in a first rotational direction 21 as indicated by arrow 21 in Fig. 3. This rotational movement brings a receiving opening 22 of a receiving chamber 23 provided within the inner ring 8 in receiving communication with an opening 24 provided in a surface or an upper wall 43 of the screw base element 6, which opening 24 is in a delivery communication with a delivery opening 25 provided in the funnel 5 for delivering a quantity of a single dose of the substance contained within the bottle 2a. The receiving chamber 23 is of such a size that it is able to only accommodate one single dose of the pharmaceutical or nutritional or confectionary substance to be dispensed by the dispensing device 3. Besides the receiving opening 22 the receiving chamber 23 comprises a discharge opening 27 which forms an opening in the circular side wall 59 of the inner ring 8. To avoid that for instance a pill 35 or a capsule being within a receiving chamber 23 passes unintentionally through the discharge opening 27 the outer ring 7 comprises a clamp 26 provided at and protruding from the inner surface of the side wall 65 of the outer ring 7. The clamp 26 is such dimensioned and sized that it is positioned in front of the discharge opening 27 when the outer ring 7 is moved in the rotational direction as indicated by the arrow 21.

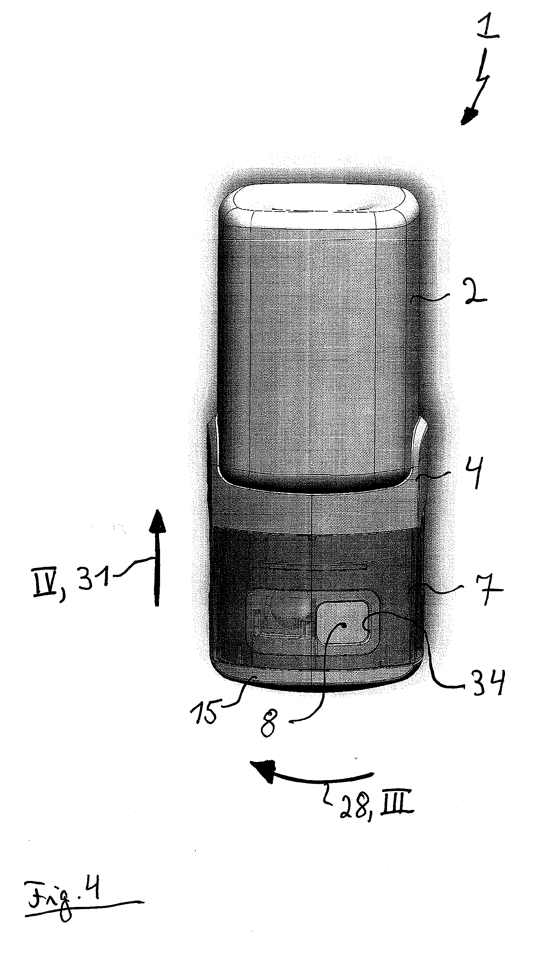

In a next step III during a dispensing cycle, the first member entity is rotated back relatively to the second member entity in a second rotational direction 28 as indicated by arrow 28 in Fig. 4. During this rotational movement, bars 58 provided at the inner and lower ring surface 57 of the inner ring 8 are sliding with their lower surface 60 along the upper surface 61 of bar like wall sections 62 provided at the screw base element 6 due to the fact that the compressed spring 12 tries to expand to its uncompressed position again and due to the fact that bar like elements 64 provided at the inner surface of the side wall 29 of the outer ring 7 are in (de)pressing engagement with side wall recesses 66 provided in the circular side wall 59 of the inner ring 8. As soon as the bars 58 come to a congruent coverage with a respectively corresponding longitudinal slot 121 provided at one end of the upper surface 61, the first member entity instantly moves up in the direction as indicated by arrow 31 in Fig. 4 and springs back into its starting position as shown in Fig. 4 and Fig. 1. The movement in this longitudinal direction is caused by the spring 12 which at this relative position of the inner ring 8 and the screw base element 6 is able to expand in its uncompressed or undepressed position again. The movement in direction of the second rotational direction 28 as indicated by the arrow 28 is supported by the spring 12 which is during the rotational movement of the first member entity relatively to the second member entity urged by a torsional load and can now expand to its starting and rest position. The clockwise reverse rotation of the outer ring 7 and the first member entity comprising the outer ring 7 in direction of the arrow 28 as shown in Fig. 4 is supported by the spring 12, which now expands back into its unstressed starting position.

Another effect of the longitudinal movement as indicated by the arrow 31 in a fourth step IV during a dispensing cycle is, that due to the longitudinal expansion of the spring 12 the PCB base 10 and the stop member 12 veer away from each other in the longitudinal direction with the effect, that stopping pins 32 provided on and protruding from a surface of the PCB 9 step out of a guiding area 33 provided in the stop member 11, in which guiding area 33 the stopping pins 32 are guided during the movement of the first member entity along the first rotational direction 21 as indicated by the arrow 21 during the second step II of a dispensing cycle. Due to the fact that no stopping pin 32 is now any longer engaged within the guiding area 33, the only mechanical connection between the PCB base 10 and the stop member 11 is provided by the spring 12.

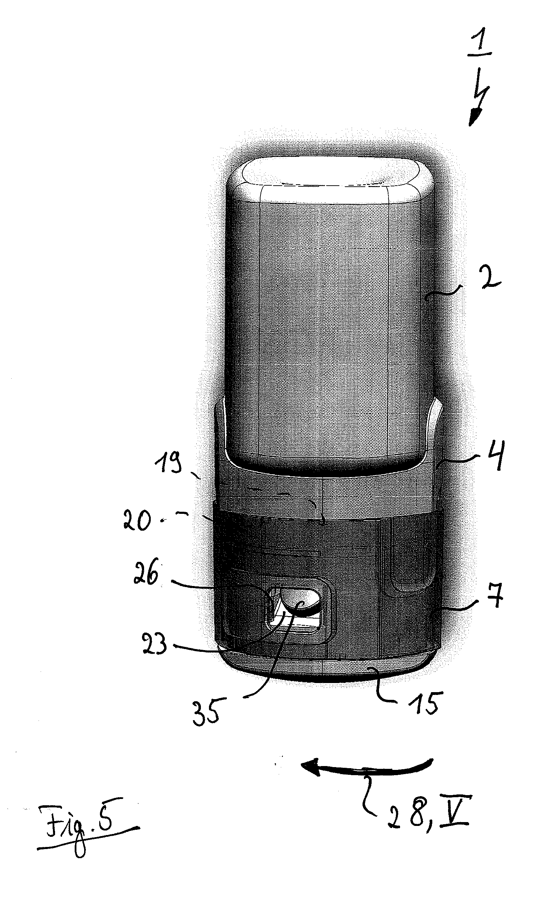

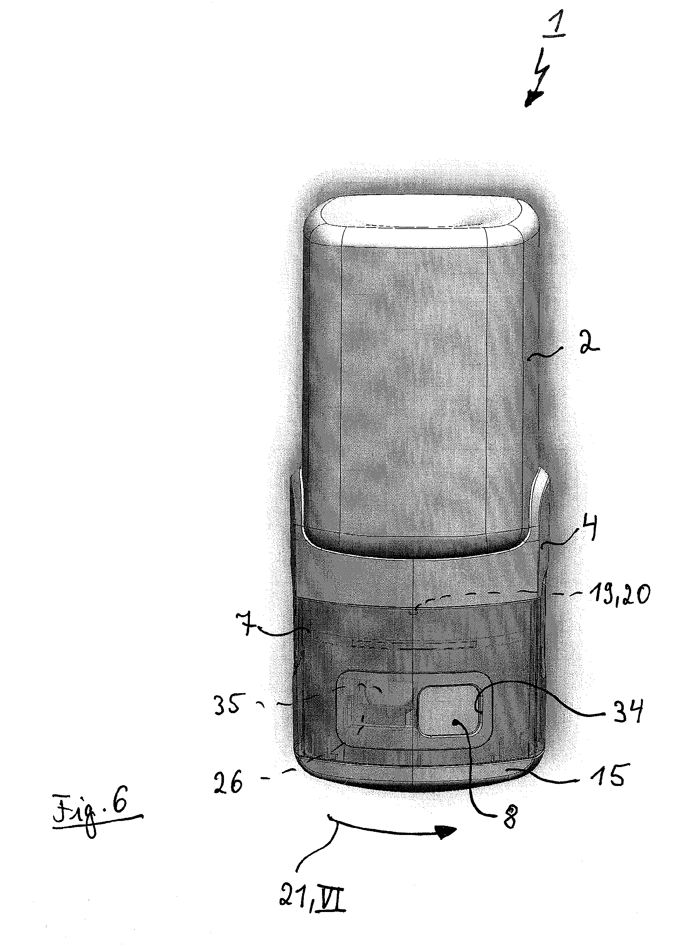

When now in a further (fifth) step V, which is a backwardly directed step, the outer ring 7 is rotated further back in a direction indicated by the arrow 28 (see now Fig. 5) the bars 58 rest in and are engaged in the longitudinal slots 121. This construction enables the outer ring 7 now to be rotated in the second rotational direction as indicated by arrow 28 in Fig. 5 as a single member whereas all the other components do not rotate together with the outer ring 7. The outer ring 7 is relatively moved and rotated in respect to all other members, parts or elements of the dispensing device 3. By rotating in the direction as indicated by arrow 28 in the step V the dispensing opening 34 or window, which is provided in the sidewall 29 of the outer ring 7 is brought into a position to lie in front of or against the discharged opening 27 of the receiving chamber 23. At this moment the substance, for instance a pill 35 will be dispensed and fall out of the dispenser 1, more precisely the dispensing device 3. Afterwards the outer ring 7 is in a last or sixth step VI of a dispensing cycle rotated back along the first rotational direction 21 as indicated by arrow 21 (see Fig. 6) until the burlings 19 come into a snap-in position with the recesses 20 again. Now the dispenser 1 or the dispensing device 3 is in a position to start a new dispensing cycle by first pulling the outer ring as indicated by arrow 18, then turning to the right as indicated by arrow 21 in Fig. 3, then turning it back to the starting position as indicated by arrow 28 in Fig. 4, then further turning it back or turning it to the left direction as indicated by arrow 28 in Fig. 5 and then finally turning it to the right again in a direction as indicated by arrow 21 in Fig. 6.

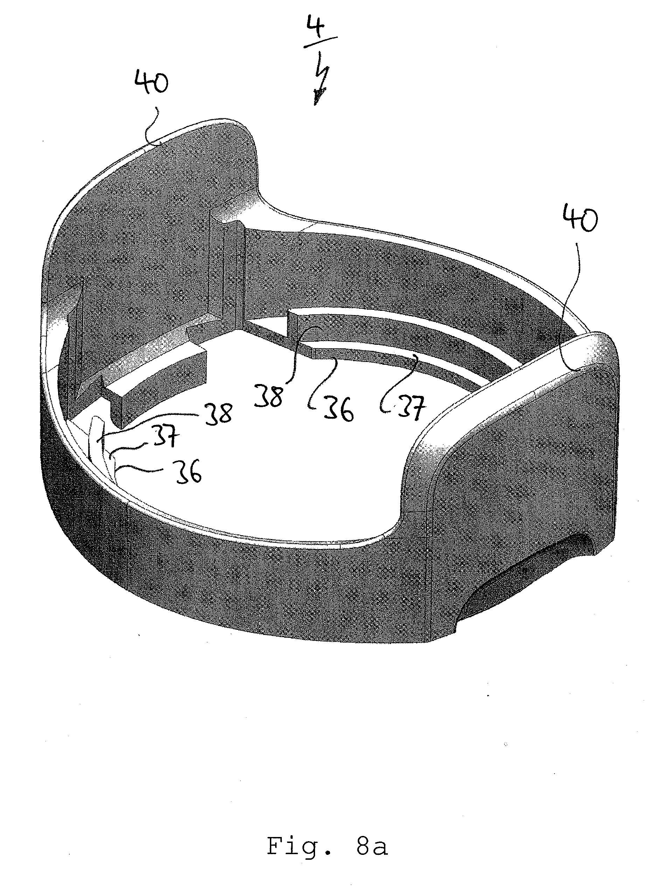

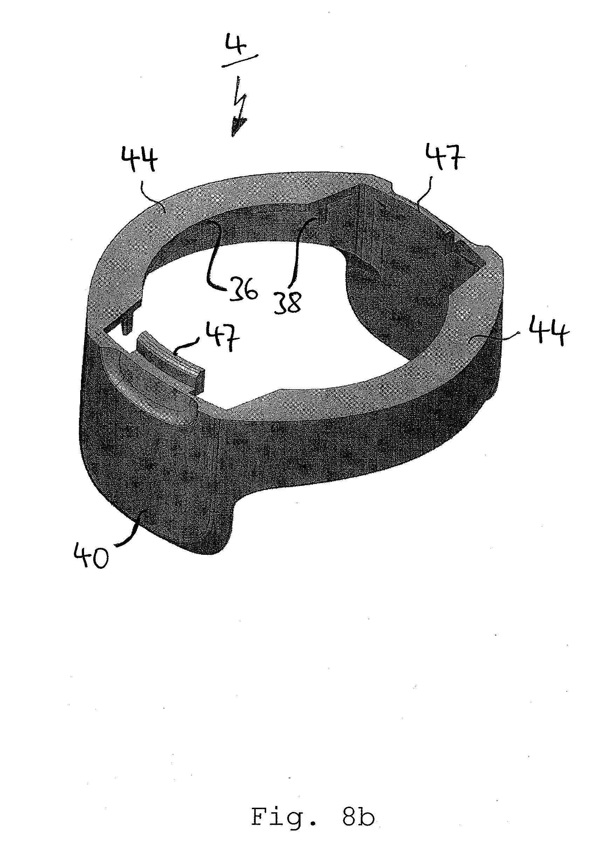

The dispensing device 3 comprises a bottle adapter 4 which is of a substantial ring form, wherein the inner diameter is defined by circular wall sections 36 which are provided with an - in respect to the positions of the parts shown in Fig. 1-6 and 8 - upper surface 37 which is respectively surrounded by a downwardly extending wall section 38. These parts 36, 37 and 38 are adapted to encompass and to engage behind a circumferential rim portion 39 provided at the outside surface of the neck 16 of the bottle 2a which is provided to be engaged with the dispensing device 3. Due to its form and comprising side portions 40 the bottle adapter 4 as disclosed by Fig. 8a and 8b is torque proof and unremovably connected to the bottle 2a when it is in its position engaging the outer circumferential rim portion 39 of the bottle 2a.

Further, the funnel 5 is arranged to lie with its ring shaped circular surface 41 on an inner circular surface 42 of the screw base element 6, wherein the delivery opening 25 of funnel 5 is in a face to face position with the opening 24 provided in the upper wall 43 of the screw base element 6. In their assembled position the sectional surface areas 44 of the bottle adapter 4 lie face to face on the circular surfaces 45 and 46 of the screw base element 6, wherein the protrusions 47 of the bottle adapter 4 engage in sections 48 provided in the outer wall 49 of the screw base element 6. Further, in their assembled condition with a bottle 2a screwed into the screw thread 50 of the screw base element 6 the upper surface of the rim portion 51 of the bottle 2a will come and be in contact with the lower surface 52 of the rim portion 53 the funnel 5. In such an assembled condition a ramp 54 is encircled and encompassed by the neck portion 16 of the bottle 2a and extends inside the neck portion 16 to be in receiving communication with the inside of the bottle 2. In the middle of the ramp 54 extents a body 55, wherein the ramp 54 and the body 55 are of such a size and such adapted to the substance contained within the bottle 2a that the substance, for instant pills 35, will be separated from each other and will be singularized so that only single doses of the solid substance are stepwise delivered to the delivery opening 25 via the ramp 54.

The inner ring 8 is with its lower circular surface 57 positioned on an upper rim surface 56 of the screw base element 6. Additionally, in this position of the outer ring 8 the bars 58 provided at a lower portion of the inner surface of the circular side wall 59 of the inner ring 8 are slideably positioned with their respective lower surface 60 on the upper surface 61 of partial bar like wall sections 62 of the screw base element 6. In order to provide a stop for a rotational movement in the first rotational direction 21 as indicated by the arrow 21, the bar like wall sections 62 are respectively provided at their respective end corresponding to the first rotational direction 21 with a stop bar 63 at which the bars 58 will abut against at the end of their respective movement step to stop further rotation.

At its outer side the circular side wall 59 of the inner ring 8 and the outer wall 49 of the screw base element 6 are surrounded and enveloped by the outer ring 7. The outer ring 7 is removably engageable with the screw base element 6 via a snapping connection constituted by the recesses 20 and the burlings 19 provided at the respective lower rim portion of the outer ring 7 and the screw base element 6. Further, bar like extending elements 64, which protrude from the inner surface of the sidewall 65 of the outer ring 7 are in a slideable engagement withl recesses 66 provided at the lower end of the outside surface of the circular sidewall 59 of the inner ring 8. The bar like elements 64 are opposing each other. At the starting point of a dispensing cycle the bar like elements 64 abut with one of their lateral faces 67 against a bordering wall of the respectively dedicated side wall recess 66.

The clamp 26 of the outer ring 7 is in the assembled position of the dispensing device 3 positioned in front of the dispensing opening 27 of the receiving chamber 23 which is composed partly by the inner ring 8 and the PCB base 10. When turning or rotating the outer ring 7 starting from a position as represented by Fig. 4 to the left side in direction of the arrow 28 to reach the dispensing position as represented by Fig. 5, the clamp 26 moves and slides in front of the receiving chamber side walls 69, 70 of the PCB base 10 as indicated by the dotted line 71. At the end of this rotational movement the clamp 26 abuts with its inner surface 72 against a side wall section 75 of the side wall 59 of the inner ring 8, which side wall section 75 borders the discharge opening 27 of the receiving chamber 23. In the opposite direction, when rotating the outer ring 7 from the position as represented by Fig. 2 during the second step II of a dispensing cycle into a right hand direction as indicated by the arrow 21 the surface 73 of the clamp 26 opposing the surface 72 abuts against the side wall section 74, which also borders the receiving chamber 23 and is positioned opposite to the wall section 75. The surface 73 additionally supports the rotation of the inner ring 8 caused by the rotation of the outer ring 7 when turning from a position as represented by Fig. 2 to a position as represented by Fig. 3.

At its top side the inner ring 8 is provided with the cap 15. The inner ring 8 and the cap 15 are connected via a snap connection constituted by several, ringlike arranged protrusions 76 protruding from the inner surface of the cap 15 and correspondingly arranged recesses 77 of the inner ring 8 adapted for engagement with the protrusions 76. Additionally the cap 15 covers the outer ring 7 with its circular surface 128. Other circular or ringlike arranged protrusions 78 may engage into corresponding recesses provided at the outside of the antenna 14 to hold it in place. Further protrusions 79, which are provided at the inner surface of the side wall 65 serve as distance pieces to the opposing outside surface of the inner ring 8. Therefore, the outer ring 7 is lying between the cap 15 and the screw base element 6 and is supported by the side wall recesses 66 of the inner ring 8, which bear the bar like elements 64. of the outer ring 7.

To hold the whole assembly together the screw base element 6 is provided with a stem 80 which extends through a central opening 81 provided in a bottom plate 82 of the inner ring 8, further extends through a central opening 83 of the PCB 9, further extends through a central opening 84 of the PCB base 10 and finally extends through the central opening 85 of the spring 12. And finally, the top of the stem 80 extends into a central opening 86 of the stop member 11, where it terminates and preferably forms a plane surface constituted by the surrounding enclosing surface of the stem receiving part 87 of the stop member 11 and the top of the stem 80. The top of the stem 80 is further provided with means 88 and corresponding sections 89 of the central opening 86, which prevent a relative rotational movement of the stop member 11 with respect to the screw base element 6. To hold this assembly together the screw 13 is screwed from above through the central opening 86 into the central opening 90 of the stem 80. Due to this arrangement and due to the spring 12, the before mentioned parts and members are held together, where nevertheless a relative movement in longitudinal direction is possible between the screw base element 6 and the stop member 11 at one side and the inner ring 7 and the outer ring 8 and the cap 15 on the other side.

The PCB 9 is arranged within the inner ring 8 resting with its lower surface 91 on posts 92 and being torque proof held by a second kind of posts 93, which are positioned within a respective corresponding insection 94 provided at the outer rim portion of the PCB 9. The lower surface 91 of the PCB 9 is equipped with some electronics 95 and energy sources 96, for instance two batteries held on the PCB 9 by holding means 97. But first of all the PCB 9 is equipped with a pair of sensors or detecting means 98 which are suitable to detect a single dose of a substance to be dispensed, for instance a pill 35, which is contained in the receiving chamber 23 and which are also suitable to detect when such a pill 35 or such a single dose of a substance leaves the receiving chamber 23 to be dispensed through the dispensing opening or dispensing orifice 34. Corresponding to the position of the detecting means 98, the sidewall parts 99 of the receiving chamber 23, which are arranged inside the inner ring 8, comprise two opposing recesses or intersections 100, which allow the detecting means 98 to have a free field of view into the receiving chamber 23 and to the respective opposing detecting means 98.

The opposing surface 101 of the PCB 9 is also equipped with electronics 95, but especially with two micro switches 102, 103 for switching on and off the electronics 95 and/or the power sources 96. With its posts and respective surfaces 104 the lower side 105 of the PCB base 10 stands on the upper surface 101 of the PCB 9. In this position, side walls 69, 70, constituting partly the receiving chamber 23 within the PCB base 10, step into the opening or section 106 provided within the PCB 9 with a depth of approximately the thickness of the PCB 9, which opening 106 also forms a part of the receiving chamber 23. Corresponding to the switch 102 is a contacting element 107 and corresponding to the switch 103 is a contacting element 108 arranged, both provided in the cover wall 109 of the PCB base 10. The surface 101 is provided with two stopping pins 32 extending therefrom and through a PCB base recess 110. To prevent relative rotational movement between a unity or entity comprising the PCB base 10 and the PCB 9 and to hold the PCB 9 at a position between the walls 69, 70 and the insection opening 106 and another unity or entity comprising the inner ring 8, the PCB base 10 is provided with a wall section 111 extending from the rim portion of the cover wall 109 and protruding in an upward direction, wherein the wall section 111 is inserted into the insection of the inner ring 8 bordered by the side wall sections 75 and 74. On its upper surface, the PCB base 10 or the cover wall 109 of the PCB base 10 comprises a grove 113 adapted for accommodating one side and one arm 114 of the spring 12. To hold the arm 14 in the grove 113, the arm is inserted under a bridge 115 provided in the cover wall 109. The bridge prevents the arm 114 from being removed out of the grove 113 in longitudinal direction of the dispensing device 3. With its opposing end and its second arm 116 the spring 12 is inserted into a grove 117 formed in the lower surface of the stop member 11.

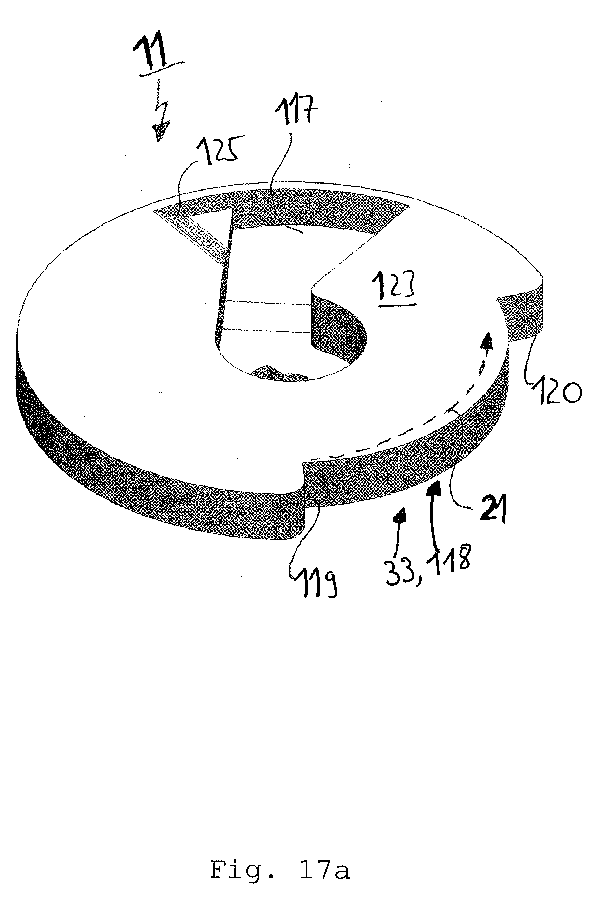

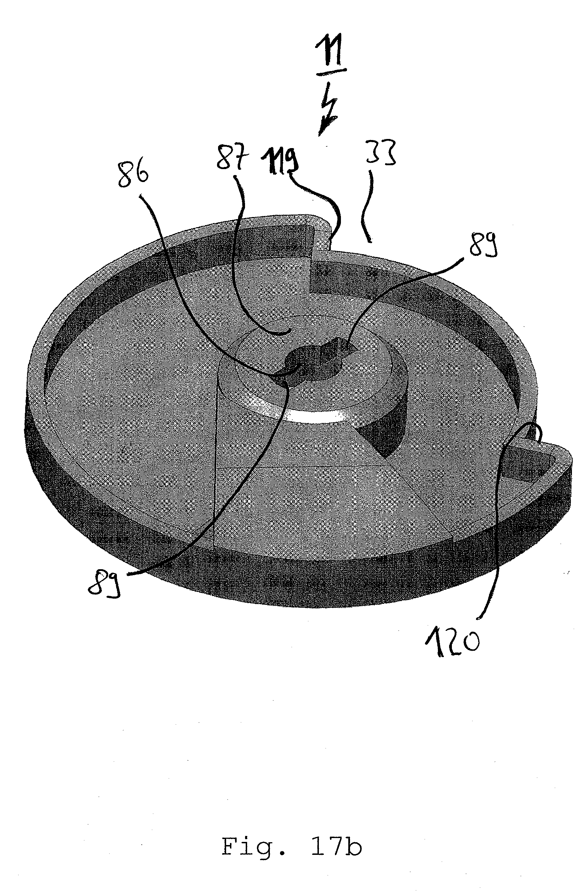

The stop member 11 further comprises the guiding area 33 formed as a recess 118 along which the stopping pins 32 are movable from a starting position with one of the stopping pins 32 being in an abutting connection with a side wall section 119 which borders the recess 118 to a stop position at which the other of the two stopping pins 32 abuts against an opposing side wall 120 bordering the recess 118. The way from the wall section 119 to the wall section 120 corresponds to the first right side or counter clockwise rotational step (step .II) of the outer ring 7 as indicated by the arrow 21 in Fig. 3 and indicated by a dotted line in Fig. 17a.

When starting a dispensing cycle, the first movement or step I in longitudinal direction as indicated by the arrow 18 in Fig. 2 causes a relative movement between the stop member 11 and the PCB base 10 holding the spring 12 in-between. Because the stop member 11 is fixedly secured to the stem 80 of the screw base element 6 this relative movement causes a compression of the spring 12. This longitudinal movement in direction of the arrow 18 is caused by pulling the outer ring 8 in the direction as indicated by the arrow 18. At this time the outer ring 7 simultaneously moves the inner ring 8 by depressing the side wall recesses 66 with its bar like elements 64. This relative movement of the outer ring 7 and the inner ring 8 with respect to the screw base element 6 causes the bars 58 provided at the lower ring surface 57 of the inner ring 8 to come free from the respectively dedicated longitudinal slots 121, provided at the outside of the screw base element 6 at an end of each bar like wall section 62 opposite to the corresponding stop bar 63 formed at the other end of a bar like wall section 62. At its other side opposite to the bar like wall section 62 each longitudinal slot 121 is delimited by an upwardly extending bar like member 122, which additionally constitutes a stop for the bar 58 against further rotational movement. As furthermore the PCB 9 is torque proof arranged within the inner ring 8 and the PCB base 10 is contained within the inner ring 8, all these parts are maintaining their positions relative to each other due to the force executed by the spring 12. When now the outer ring 8 is pulled in the direction as indicated by the arrow 18, the unity constituted by the members 7, 8, 9 and 10 is moved towards the stop member 11 and the surface of the cover wall 109 comes to lie on the surface 123 of the stop member 11. This causes the contact element 107, which now comes into contact with the surface 123, to be moved downwardly and to switch the switch 102 to its "on" position. The second contact element 108 is not moved at this time because it is arranged within the grove 117 so that there exists no contact to the surface 123.

In a next step (step II) of the dispensing cycle the outer ring 7 is rotated in the first rotational direction as indicated by the arrow 21 in Fig. 3. This rotational movement goes along with a likewise rotational movement of the inner ring 8 due to the contact of the side face 67 of the respective bar like elements 64 with a respectively dedicated bordering bar 68 of a side wall recess 66 and of a contact of a surface section 124 of the clamp 26 with a wall section 74 of the outer ring 7. As additionally the PCB 9 is via posts 93 operating in cooperation with in sections 94 inserted in the inner ring 8 in a torque proof condition and as at the same time furthermore the PCB base 10 is torque proof inserted in the insection opening 106 of the PCB 9 via its receiving chamber side walls 69, 70, the first member entity is rotated relatively to the stop member 11, causing the stopping pins 32 to move from the wall section 119 to the wall section 120. Caused by this relative movement between the PCB base 10 and the stop member 11, the contact element 108 comes out of the groove 117 and into contact with the lower surface 123 of the stop member 11 when passing a ramp 125. Passing the ramp 125 and now resting on the surface 123 causes a moving of the contact element 108 in a downward direction contrary to the direction indicated by the arrow 18. Due to this movement the lever of the on/off-switch 103 is operated and switched. This rotational movement is finished and stopped by a mechanical stop provided by a stopping pin 32 butting against the wall section 120 and the bars 58 butting against a corresponding stop bar 63 of the screw base element 6, respectively.

In a next third step III, the first member entity is reversely rotated in a second rotational direction 28 as indicated by arrow 28 in Fig. 4 until it returns in its starting position as shown in Fig. 2. This position is reached when a stopper pin 32 abuts against the wall section 119 of the stop member 11 and the bars 58 abut against a corresponding bar like member 122 delimiting a respective slot 121 provided at the screw base element 6. During this reverse back rotation the contact element 108 again passes the ramp 125 and enters into the groove 117 resulting in now moving in a downward direction as indicated by arrow 18, which causes the contact element 108 to come free from the lever of the on/off-switch 103 such, that an on/off-switching cycle of the on/off-switch 103 is finished to its closed off-position, wherein the on-switch position will be started when the contact element 108 passes in forward rotational direction over the ramp 125 when rotating in the first rotational direction as indicated by arrow 21.

When reaching the slots 121 the bars 58 leave the upper surface 61 of the bar like wall sections 62 so that immediately the spring 12 may expand from its compressed condition and urges the bars 58 into the slots 121, causing in a fourth step IV an upwardly directed movement of the first member entity as indicated by the arrow 31. At the same time the burlings 19 and the recesses 20 come into a snapping position. Simultaneously, the surface of the cover wall 109 of the PCB base 10 removes itself from the surface 123 of the stop member 11 when moving as a part of the first member entity in the direction as indicated by arrow 31. This further immediately causes the contact element 107 to come free from the lower surface 123 and the depression force caused by this surface, so that the lever of the on/off- switch 102 being in a contact therewith comes free from the contact element 107 and switches back into its off-position. These on/off-switching cycles of the on/off-switches 102, 103 may be used for switching on/off the electronics 95, 95a, the sensors 98, the power sources 96 etc.

When now for finishing a dispensing cycle in a fifth step V the outer ring 7 is rotationally moved further in a clockwise direction as indicated by arrow 28 in Fig. 5, it is only the outer ring 7 which rotates. The inner ring 8 is hindered to rotate further in this direction by its bars 58, which now lie against the bar like members 122 providing a stop. When rotating the outer ring 7 in the direction as indicated by arrow 28 as shown in Fig. 5, the snap-in connections of burlings 19 and recesses 20 come free again to allow the outer ring 7 to be rotated between and held by the cap 15 and the upper surface 44 of the bottle adapter 4. This rotational movement of the outer ring 7 is stopped when the bar like element 64 abuts with its second side face 126 against a respective second bordering bar 127 provided at the other end of a respective side wall recess 66, which other end is opposite to the end delimited by a respective bordering bar 68. Another stop is provided by a stopping contact between the inner surface 72 of the clamp 26 and the side wall section 75 of the inner ring 7. When this stopping position has been reached, the single dose of a substance, for instance a pill 35, contained in the receiving chamber 23, will be dispensed and falls out by gravity because the receiving chamber 23 and its discharge opening 27 are then in a congruent overlapping position with the dispensing orifice or dispensing opening 34 being provided at the outer ring 7. The discharging of the substance or pill 35 from the receiving chamber 23 will be detected by the sensors or detecting means 98 provided on the PCB 9. Afterwards, to finish a dispensing cycle of a single dose of a substance contained in a bottle 2a the outer ring 7 is finally in a sixth step VI turned back into its starting position along the first rotational direction 21 as shown in Fig. 6. Having reached the starting position again, the clamp 26 is then positioned to lie in front of the receiving chamber 23, wherein the dispensing orifice or opening 34 is closed by the circular side wall 59 of the inner ring 8.

This rest position is appreciably indicated by a snapp-in-connection of the burlings 19 and the recesses 20.

The electronics 95, 95a or the printed circuit board (PCB) 9 as such may comprise a memory chip or an electronic circuitry or an electronic circuit 2500, which allows to store and transfer the dispense information to an external device via Bluetooth or RFID (radio frequency identification) or other wireless communication. Therefore, the antenna 14 is preferably a RFID antenna.

With respect to the material of which the dispensing device 3 or the reservoir or bottle 2a are formed, it is most preferably that they are formed of thermoplastic resins. Especially, they may be made of HDPE (high density polyethylene) or PP (polypropylene) and may contain or may be made of a desiccant.

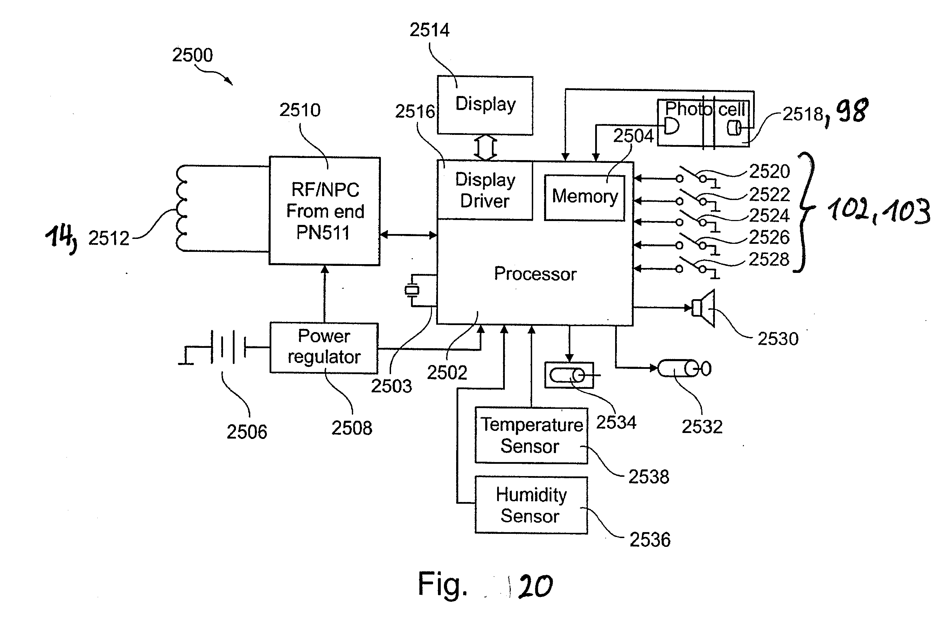

FIG. 20 shows a block diagram of an electronic circuit 2500, which is incorporated in the PCB 9 and/or the electronics 95,95a for use in monitoring and controlling administration of medications and for communication. The electronic circuit 2500 includes a processor 2502, which may, for example, be an 8-bit microcontroller, such as a P89LPC936, developed by Philips and available from NXP Semiconductors Netherlands B.V. Of course, it will be understood that other processors or microcontrollers may also be used in the electronic circuit 2500. In some embodiments, the processor 2502 may be clocked by an external clock or crystal 2503.

The processor 2502 may include a memory 2504, for storing programmed instructions for the processor 2502 and/or data used by the electronic circuit 2500. Alternatively, the memory 2504 may include one or more external memory devices (not shown). The memory 2504 may include non-volatile memory, such as flash memory, EEPROM memory, or static memory, and/or volatile memory, such as DRAM.

The electronic circuit 2500 is powered by an energy source 96 in the form of a battery 2506, which may have its electrical characteristics adapted to the needs of the electronic circuit 2500 by a power regulator 2508. The battery 2506 may be a conventional replaceable battery, or a rechargeable battery, which may be recharged, for example, when the device is connected to a docking station (see below). The power regulator 2508 may also include the ability to detect the status of the battery 2506, and provide the status information to the processor 2502. In some embodiments, an additional battery measuring device (not shown) may be used to measure the status of the battery 2506.

The processor 2502 communicates with a first transceiver 2510, which communicates wirelessly with other electronic devices via a first antenna 2512 and/or antenna 14. The first transceiver 2510 uses radio frequency (RF)-based communication, such as Near Field Communication (NFC) or other wireless communication technologies suitable for short-range and/or low-power wireless communication, such as other RFID technologies, Bluetooth, or ZigBee. Whereas power considerations permit longer range communications, other wireless communications technologies, such as Wi-Fi, WiMAX, or various cellular technologies may be employed.

In some embodiments, where the device includes an optional display 2514, preferably provided at the outer ring 7 or the cap 15, the processor 2502 may further include a display driver 2516, to operate the display 2514. In some embodiments, the display driver 2516 may be implemented at least in part as driver software. In some embodiments, the display driver 2516 may be a separate device (not shown), rather than being included in the processor 2502.

To constitute the at least one detecting means 98 for detecting and/or monitoring the dispense of a solid substance or pill 35, an optical detector 2518, such as a laser detector, an LED and detector, an infrared LED and detector, or other opto-electronic detection device, is used to determine when a solid preparation, such as a pill 35, has been dispensed by the device. A signal from the optical detector 2518 is provided to the processor 2502 for evaluation. In some embodiments, where there are multiple optical detectors, signals from each of these optical detectors are provided to the processor 2502.

The electronic circuit 2500 may also include numerous control switches 102, 103 for adjusting the settings of the processor 2502 and/or of the electronic circuit 2500. For example, a first switch 2520 can be used to activate or deactivate the electronic monitoring and communication. A second switch 2522 can be used to detect tampering with the dispenser device by detection its removal from the reservoir or bottle 2. Third and fourth switches 2524 and 2526 may be used, for example, for setting the time in hours (third switch 2524) and minutes (fourth switch 2526). A fifth switch 2528 may be used to select use of an audible alarm, such as a buzzer 2530, which may be sounded when, for example, a patient has forgotten to dispense a medication at the time that he is supposed to take it, or when temperature and/or humidity sensors indicate that the medication is being improperly stored. It will be understood that, depending on the user interface needs of the embodiment, the control switches may be assigned to other functions.

The electronic circuit 2500 may also optionally include alarm devices, such as the buzzer 2530 and/or a vibration device 2532. These alarm devices may be activated by the processor 2502 separately or in unison, to alert a user to a variety of conditions. In some embodiments, the buzzer 2530 may be controlled to produce a variety of different sounds to alert a user to various conditions. For example, one sound may be used to warn the user that he should take a dose of a medication, another sound may be used to warn the user that he is attempting to dispense additional medication before it is safe to take another dose, a further sound may be used to warn the user that the medication in the container is running low, another sound may be used to indicate that the battery is low, and still another sound may be used to indicate to the user that temperature and/or humidity sensors are indicating that the medication is not being properly stored. In some embodiments, the buzzer 2530 may be a small speaker, capable of producing sounds including buzzing noises, speech, musical tones, or other sounds, depending on the message to be conveyed to the user.

The timing of the sounds or vibrations produced by the buzzer 2530 and/or vibration device 2532 may be controlled by the processor 2502 to convey particular meanings or warnings. For example, the processor 2502 may be programmed to use the buzzer 2530 to produce a warning sound at a predetermined time after the solid preparation should have been dispensed. For example, a short beep could be generated every minute during one hour following the time when the solid preparation should have been dispensed. The processor 2502 may be programmed to cease such warnings when the solid preparation has been dispensed through the dispenser.

The processor 2502 may be programmed to use the vibration device 2532 in a similar manner. For example, the vibration device 2532 may be switched on for one second at the time that the solid preparation should be dispensed. This may be repeated, for example, sixty minutes later, if the solid preparation is not dispensed.

In some embodiments, the electronic circuit 2500 may optionally be connected to a blocking mechanism 2534. When activated, the blocking mechanism 2534 prevents the dispenser from dispensing a solid preparation. This can be achieved, for example, by sending electrical signals to a motor or solenoid to move a stopper notch between a locked and an unlocked position, as described above. The blocking mechanism 2534 may be used, for example, to prevent a user from dispensing a further dose of a medication during a time period over which a further dose is not needed or could be dangerous, or from dispensing medication which may have been damaged by exposure to temperatures or humidity levels outside of an acceptable range.

In some embodiments, the electronic circuit 2500 may further include additional sensors, such as an optional humidity sensor 2536 and/or an optional temperature sensor 2538, positioned in such a way that they are able to detect the humidity and/or temperature of the pills, capsules, or other solid preparations stored in the dispenser device. The humidity sensor 2536 may, for example, be a capacitive humidity sensor, a resistive humidity sensor, a thermal conductivity humidity sensor, or other suitably small, commercially available electronic humidity detection device. Similarly, the temperature sensor 2538 may be a thermistor or other resistance temperature detector, or any other suitably small, commercially available electronic temperature detector. These sensors should be positioned so that they measure the temperature and/or humidity of the pills, capsules, or other solid preparations stored in the container, bottle, or dispenser.

Generally, the electronic circuit 2500 may be configured to fit within a portion of the dispenser mechanism, as shown in earlier figures above. Individual components of the electronic circuit 2500 may be built into other portions of the dispenser, depending on their function. For example, the humidity detector 2536 and temperature detector 2538 may be positioned so that they measure the humidity and/or temperature in the locations where pills, capsules, or other solid preparations are stored.

In addition to the features described above, the dispenser mechanism may include an identity detection device (not shown). Such an identity detection device may be connected to the electronic circuit 2500 to permit the user of the dispenser to be identified. An example of such an identity detection device is a fingerprint reader and identifier. The processor 2502 may be programmed to accept signals from such a fingerprint reader (not shown), and to activate the dispenser only when the fingerprint read by the fingerprint reader matches a stored fingerprint. The stored fingerprint may be stored in the memory 2504, or in a memory associated with the fingerprint reader (not shown). Since fingerprints are unique, the fingerprint of the authorized user of the device may be stored, so that only the authorized user of the device is able to activate the dispenser and to dispense a solid preparation. Other identity detection devices could also be used, including other (preferably small/portable) biometric devices, or security measures such as requiring the user to enter a combination or a personal identification number (PIN).

By adding such an identity detection device to the electronic circuit 2500, an identity function may be implemented for the dispenser. This identity function makes it possible that only an authorized or intended user, such as the patient or a caregiver, can activate the dispenser. Using this feature, the solid preparation could not be dispensed, for example, by a child who finds the device. Additionally, the identity function may reduce the risk of taking the wrong medication, for example if there are several such dispensers being used by different people in a single household.

Further, the electronic circuit 2500 may be connected to an RFID reader (not shown) in the dispenser. Some medication containers, reservoirs or bottles 2a (not shown) may be equipped with RFID chips (not shown) that can contain information on the medication in the container. Such RFID chips may be placed on or built into the container when the container is manufactured, or at a later time, such as when a pharmacist provides the container containing medication to the patient. The RFID chip in such a container may be a standard MIFARE RFID chip, or any other type of RFID chip or tag. A drug manufacturer, physician, and/or pharmacist may store information on the RFID chip. For example, the RFID chip may include the date and time of packing a medication in the container, the content of the container, the drug type and number of pills, the expiration date of the medication, a unique identification number, patient medication intake times, length of the course of treatment, pharmacist license number, prescribing physician license number, proper storage temperature and humidity ranges, and/or other information pertaining to the solid preparation contained in the container.

The RFID reader may be used to read this information from the RFID chip attached to the container when the dispenser is attached to the container. The information can then optionally be stored in the memory 2504, and used by the processor 2502 for a variety of purposes. For example, if the dispenser includes a display, such as the display 2514, the information read by the RFID reader may be displayed. This can reduce the risk of taking the wrong medication or medication that has passed its "use by" date. The risk of taking the wrong medication may be especially pronounced when a patient needs to take two or more types of medication. When an RFID reader in the dispenser is used with an RFID chip on the container, the patient is able to read on the display which of his medications is contained in the container. This may be particularly useful when the labelling of the container has faded, for example due to frequent use or contact with water or solvents. The processor 2502 may use the information read from an RFID chip on the container for purposes such as displaying the drug contained in the container, determining when the container is almost empty (based on pill count), automatically programming the times that the solid preparation should be dispensed or accessed so that the processor 2502 may generate alarms at the proper times, producing a warning when a medication has expired or has been stored at an unacceptable temperature and/or humidity level, preventing a user from dispensing or accessing the solid preparation at the wrong times, after it has expired, after a course of treatment has been completed, if improper temperature and/or humidity conditions may have affected the medication, and other uses for such information.

Additionally, in some embodiments, the RFID reader may also store information back into a RFID chip provided on the container or reservoir 2. This means that compliance information may be available in the container chip when it is returned to the pharmacist, for example for a refill.

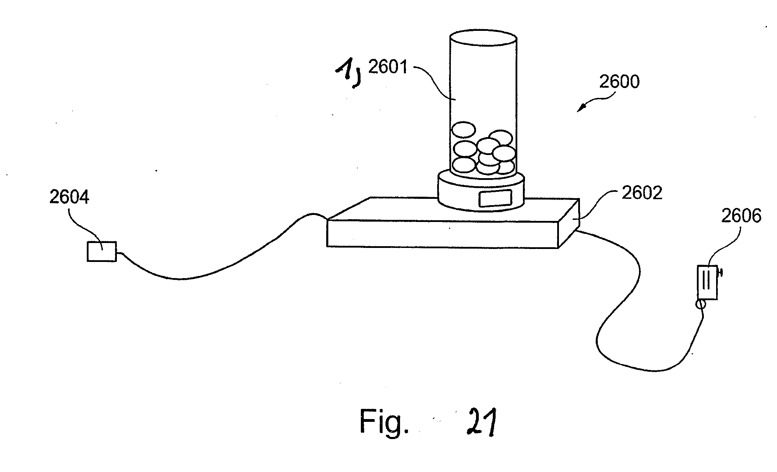

Referring now to Fig. 21, a system 2600 for communicating with a dispenser 2601 provided with a dispensing device 3 is described. A docking station 2602 is used for electronic data communication and electronic data transfer between the dispenser and a computer (not shown) or other communication device (not shown). Additionally, in some embodiments, the docking station 2602 may be used to recharge a rechargeable battery in the dispenser.

In some embodiments, the docking station 2602 may include a wired connection 2604, such as a USB connection or other wired connection for transferring data between the docking station 2602 and a computer or other communication device. In some embodiments, the docking station 2602 may include a wireless communication device (not shown) to allow the docking station 2602 to communicate via a wireless connection, such as through a cellular network, a wireless wide area network, or a wireless local area network. The docking station may be powered using an AC mains adapter 2606, or through power received over the wired connection 2604.

The docking station 2602 is also equipped with an electronic reader-writer device (described below), for reading and writing data from the dispenser. In some embodiments, where the dispenser is able to communicate directly with a wide area network or cellular network, or where the communication is handled by a portable reader, such as a mobile phone equipped with an NFC reader, the docking station 2602 may not be needed for the dispenser to communicate its data.

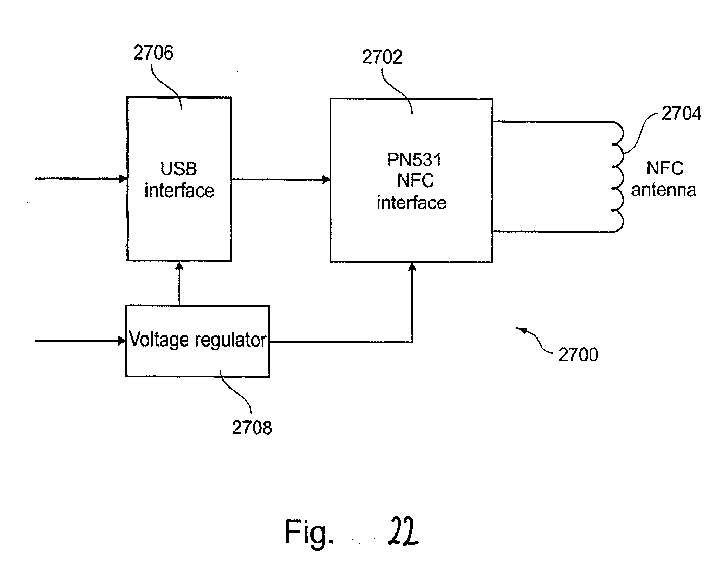

Fig. 22 shows a block diagram of an electronic reader-writer device 2700 suitable for use in the docking station 2602 of Fig 21. The reader-writer device 2700 includes a second transceiver 2702 with a second antenna 2704 for communicating with the first transceiver 2510 in the dispenser, as shown in FIG. 20. When used with the reader-writer device 2700 in a docking station, the dispenser may preferably use a low power, short range communication technology, such as Near Field Communication (NFC), Bluetooth, or ZigBee. Other communications technologies suitable for longer range wireless communications may also be used, such as WiFi or other wireless local area network (WLAN) technology. Of course, the communication technology used by the reader-writer should be compatible with the communication technology used by the dispenser. Alternatively, a physical electrical connection between the dispenser and the docking station could be used, assuming that the dispenser includes an appropriate interface. For example, if the dispenser has a USB interface, it may be possible to connect it to the docking station (or directly to a USB-equipped external computer) using the USB interface. A physical interface, such as a USB interface, may also be useful for charging a rechargeable battery in the dispenser.

The reader-writer device 2700 also may include a wired connection interface 2706. The wired connection interface 2706 may be, for example, a USB interface through which the reader-writer device connects the docking station to an external computer system. Other types of wired connections, such as a serial connection or a wired Ethernet connection could also be used.

The reader-writer device 2700 may be powered from an AC adapter (not shown) through a voltage regulator 2708. Alternatively power may be received from other sources, such as through the wired connection interface 2706.

Once the data are transferred from the dispenser to an external computer (through a docking station, such as the docking station 2602, shown in Fig. 21, when the dispenser is unable to communicate directly with the external computer), the external computer can transfer the data to a remote computer via a wide area network, such as the Internet. The dispenser may also receive data via a wide area network through an external computer (and, possibly a docking station). Further, programming or instructions for the electronic circuit 2500 of the dispenser 1 or dispensing device 3, as shown in Fig. 20, may be sent from a computer at a remote location, and communicated to the dispenser 1 or dispensing device 3 via the Internet or other wide area network. The remote computer may, for example, be accessible by a physician, pharmacist, or other medical professional who is overseeing the therapy compliance of the patient who is using the dispenser. It will be understood that in some embodiments, where the dispenser 1 or dispensing device 3 includes wide-area networking or cellular communication capabilities, the dispenser 1 or dispensing device 3 may be able to connect to the Internet and/or the remote computer system without using an external computer or docking station to establish the connection. It will also be understood that in some embodiments, a mobile device, such as an NFC-equipped mobile telephone may be used to communicate between the remote computer and the dispenser 1 or dispensing device 3.

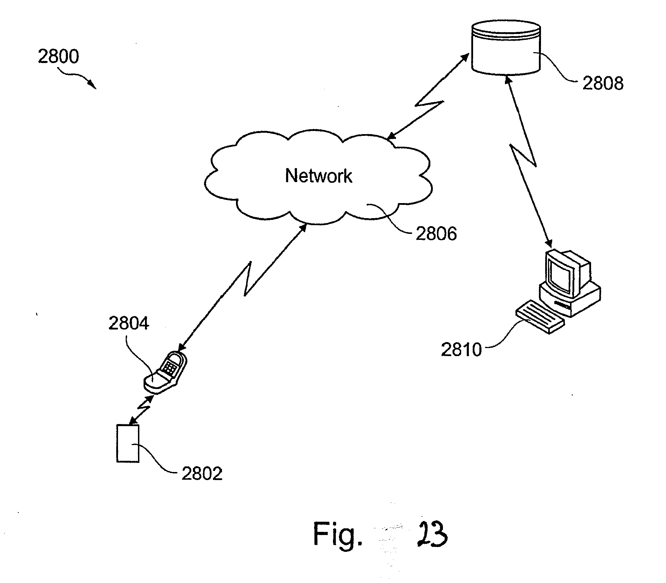

Such a system or means is shown in Fig. 23. The system or therapy compliance monitoring communication means 2800 of Fig. 23 includes one or more dispensers 2802 provided with a dispensing device 3, which include the electronic circuitry 2500 as shown in Fig. 20. For purposes of illustration, these dispensers include NFC communication circuitry, which allows them to transfer data between a dispenser 2802 and an NFC-equipped mobile telephone 2804. The NFC-equipped telephone 2804 can wirelessly communicate via a wide area network 2806, such as a cellular communication network or the Internet with a remote database 2808, which collects and stores information from the dispenser(s) 2802. The remote database 2808 can be accessed (through the wide area network 2806 or a different wide area network) by a remote computer 2810, which may also remotely send instructions to the disenser(s) 2802 through the wide area network 2806 and the mobile telephone 2804.

It will be understood that the communication path may be somewhat different, depending on the technology used. For example, if no NFC-equipped mobile phone is available, the dispenser may use a docking station (not shown) connected to a computer (not shown) to communicate with the remote database 2808 and/or the remote computer 2810. Alternatively, in some embodiments, the dispenser may be able to directly connect to the wide area network, and communicate with the remote database 2808 and/or the remote computer 2810 without using an NFC-equipped mobile telephone or a docking station.

As can be seen in Fig. 23, the therapy compliance monitoring and communication means provided can be mobile. The dispenser 2802 is arranged for monitoring the therapy compliance of a patient, and for remotely allowing or disabling the dispensing of a solid preparation, to help ensure therapy compliance. Wired and/or wireless communications can be used to report therapy compliance to the remote computer 2810, which may be used by a physician, pharmacist, or other medical caregivers to monitor compliance. Additionally, administration of therapy may be controlled or adjusted from the remote computer 2810, depending on the therapy compliance reporting received. Further, as can be seen, in addition to the Internet, other communication technologies may be used in the remote surveillance and control of therapy compliance, including mobile platforms, such as the mobile telephone 2804, and the like.

When a container with a solid preparation is issued by a pharmacist, the dispenser 2802 according to an embodiment of the invention is put in place on the container, such that solid preparations may be taken from the container by pushing a button on the dispenser 2802. The dispenser 2802 has a built-in clock/calendar so that when the button is operated and a solid preparation, such as a pill 35 or tablet is dispensed, the date and time of this event are stored in a memory in the dispenser 2802. Similarly, the times of other events, such as recording of temperature and or humidity readings can be stored. The dispenser 2802 may optionally be programmed so that the solid preparation may only be dispensed at pre-programmed times, depending on the medication prescribed, and the instructions of the physician and/or pharmacist. This may prevent a patient from taking too many doses, since the dispenser 2802 is blocked after a dosage is taken, and will only dispense a further dose when the next dosage should be taken. It should also be noted that this option of programming the dispenser provides the opportunity to register and regulate a combination therapy, whereby more than one type of medication must be taken, as will be described in greater detail below.

Next, the date and time stamp at which a dose was dispensed, and/or other information, such as temperature and/or humidity data are transferred over the wide area network, which may be a mobile telephone network, such as GSM or GPRS, to the patient's record in the remote database 2808. As shown in the figure, in some embodiments, this transfer may be accomplished by reading the data from the dispenser using a Near Field Communication (NFC) mobile phone 2804, or by using another gateway for conversion of data from NFC or Bluetooth devices into SMS and GPRS data. It will be understood that other communication options, as described above are also possible. In some embodiments, other data collected by the dispenser may also be transferred along with the compliance data. For example, data concerning the temperature and humidity of the stored medication may be transferred. These data may indicate whether the pills, capsules, or other solid preparations are being stored in appropriate conditions. This information may be used to send the patient and/or the pharmacist or physician a warning if the medication is being stored at an inappropriate temperature or humidity. This data could also be used, for example, to dynamically adjust the expiration date of a medication, depending on its storage conditions, or to prevent a patient from taking medications which could become dangerous if stored for a period of time in an inappropriate manner. Other information, such as the battery status may also be transferred. This data may, for example, be used to warn the patient or pharmacist if a non-rechargeable battery in the dispenser device will need to be replaced.

The patient record in the database 2808 contains various kinds of patient information, including the therapy compliance records for the patient received from one or more dispensers. This information can be securely accessed by physicians, pharmacists, or other authorized medical caregivers from a remote computer 2810 over a wide area network, such as the Internet. The compliance data may be correlated and analyzed in the remote database 2808 or on the remote computer 2810, and if mal-compliance or non-compliance are detected, the patient can be warned, for example via an SMS service or the like. In some embodiments, when non-compliance or mal-compliance are detected, a call centre, pharmacist, and/or care organization may receive an instruction to call the patient to discuss his mal- or non-compliance.

A dispenser 1 or dispensing device 3 according to an embodiment of the invention can also communicate with another dispenser, either directly, through a docking station, or through a network. An advantage of such a dispenser is that it is possible to regulate an order in which two or more medications are taken. For example, in AIDS treatment, a combination of drugs may be prescribed, which need to be taken in a strict order and according to a strict time schedule. For example, the prescription schedule can specify that a first medication should be taken first, followed within one hour by a second medication. If the patient forgets that he has already taken the required dosage of the first medication, he may try to "correct" this by taking another dose of the first medication. Such non-compliance can have serious effects on the health of the patient and on the effectiveness of the treatment.

By communicating with each other, the dispensers according to an embodiment of the invention can reduce this problem. Using the above-described example, a first dispenser for a container containing the first medication can block further dispensing of the first medication until it receives a communication indicating that a second dispenser for a container containing the second medication has dispensed a dose of the second medication. Thus, a new dose of the first medication can only be taken after the required dose of the second medication has been taken. The time for taking the second medication can be set by the second dispenser receiving a communication indicating that the first medication has been dispensed by the first dispenser, causing the second dispenser to set a buzzer or other alarm feature to provide a warning one hour later that the second medication should be taken. By use of dispensers that are able to communicate with each other, according to an embodiment of the invention, a strict medication regime can be followed with reduced effort by the patient and with an increased rate of compliance.

It should be noted that in accordance with various embodiments of the invention, communication between the dispensers can be achieved directly between the dispensers, or via an indirect method. For example, the dispensers may communicate through a base station, or through a wireless network. Also, the dispensers could communicate indirectly through a database, such as the remote database 2808 shown in Fig. 23, or through another computer or communication device that receives and sends communications to dispensers in accordance with various embodiments of the invention.

A method of monitoring a patient's medication compliance can comprise
a. providing a dispenser system comprising a dispenser 1 and/or a dispensing device 3, which dispenser system tracks at least one type of patient medicine compliance data, said dispenser system being connected to a container,
b. providing a wave energy transmitter and a power source to drive said transmitter for transmission of said patient's medicine compliance data to a remote location, said transmitter being electronically connected to said dispenser system for said transmission and said data transmitter and power source being connected to said container,
c. providing a receiver at said remote location and providing a computer to which said receiver inputs patient compliance data,
d. having either said dispenser system or said computer programmed to store the prescribed medicine dosage and regime of said container,
e. having either said device or said computer programmed to calculate compliance requirements for each dosage administration for the prescription period of the medicine and for comparing the actual medicine consumption or container usage with the compliance requirements.

Preferably a dispenser system as described above is used for carrying out the invention. For this purpose further especially a container is used with an absorber function obtained by an absorber component.

## Claims

1. Dispensing device (3), especially a medical dispensing device, comprising an inner ring (8) provided with a receiving chamber (23) having a receiving opening (22) and a discharge opening (27), especially formed in a circular side wall (59) of the inner ring (8) and an outer ring (7) having a dispensing opening (34), especially formed in a side wall (65) of the outer ring (7), wherein the inner ring (8) and the outer ring (7) are rotatable arranged at a screw base element (6) having an opening (24) for delivering a substance therethrough, which substance is contained in a reservoir (2) which is detachably attachable to the dispensing device (3) to form a dispenser (1),
**characterized in,**
**that** in a first condition of arrangement the inner ring (8) and the outer ring (7) are engaged to each other for commonly performing a first rotational movement relative to the screw base element (6),
which first rotational movement comprises
a first step (I) of commonly releasing the inner ring (8) and the outer ring (7) from a common starting position where said inner ring (8) is in a locked position with the screw base element (6) and
a second step (II) of commonly rotating the inner ring (8) and the outer ring (7) along a first rotational direction (21) into a first stop position, where the receiving opening (22) of the receiving chamber (23) comes into a receiving communication with the opening (24) provided in the screw base element (6) and
a third step (III) of commonly rotating the inner ring (8) and the outer ring (7) back into the starting position of the second step (II) along a second rotational direction (28) opposite to the first rotational direction (21) and
a fourth step (IV) of commonly bringing the inner ring (8) and the outer ring (7) back into their common starting position,
and **that** in a second condition of arrangement the inner ring (8) and the screw base element (6) are engaged to each other for allowing the outer ring (7) to be rotated relatively with respect to the inner ring (8) and the screw base element (6) during a second rotational movement,
which second rotational movement comprises a fifth step (V) of rotating the outer ring (7) further along the second rotational direction (28) starting from the starting position into a second stop position, where the discharge opening (27) of the receiving chamber (23) is in dispensing communication with the dispensing opening (34) of the outer ring (7) and
a sixth step (VI) of rotating the outer ring (7) along the first rotational direction (21) back into the starting position.

2. Dispensing device (3) according to claim 1, **characterized in, that** it comprises a funnel (5), which funnel (5) has a circular and inclined ramp (54) which is such positioned with respect to a neck (16) of the reservoir (2) and such designed and dimensioned with respect to the dimensions of the substance or pill (35) that it allows to singularize a substance or pill (35) from a plurality of substances or pills (35) contained in the reservoir (2) and to deliver it in a single dose manner to the opening (24) provided in the screw base element (6).

3. Dispensing device (3) according to claim 1 or 2, **characterized in, that** it comprises a printed circuit board (9), which is torque proof arranged within the inner ring (8) to be movable together with the inner ring (8) in rotational directions and relatively with respect to a stop member (11), which stop member (11) is torque proof connected to the screw base element (6).

4. Dispensing device (3) according to claim 3, **characterized in, that** it comprises a printed circuit board base (10), which is torque proof arranged within the inner ring (8) at a position between the printed circuit board (9) and the stop member (5) and to be movable together with the inner ring (8) and the printed circuit board (9) in rotational directions and relatively with respect to a stop member (11).

5. Dispensing device (3) according to claim 3 or 4, **characterized in, that** the printed circuit board (9) is provided with at least one contact element (107, 108) which contact element (107, 108), preferably on its surface facing the printed circuit board (9), interacts with at least one switch (102, 103) provided at the printed circuit board (9) and which contact element (107, 108), preferably on its surface facing a surface (123) of the stop member (11), interacts with this surface (123) of the stop member (11) and/or a groove (125) or cavity provided therein, to be switched during rotational movement of the inner ring (8) in the first rotational direction (21) and/or the second rotational direction (28).

6. Dispensing device (3) according to one of the preceding claims, **characterized in, that** the dispensing device (3), especially the printed circuit board (9) and/or the inner ring (8), is/are provided with electronics (95, 95a) and/or at least one energy source and/or at least one detecting means (98) detecting and/or monitoring the dispense of a substance or pill (35) from the dispenser (1) and/or at least one humidity sensor and/or at least one temperature sensor.

7. Dispensing device (3) according to claim 6, **characterized in, that** the electronics (95, 95a) and/or the at least one energy source and/or the at least one detecting means (98) and /or the at least one humidity sensor and/or the at least one temperature sensor are switched on/off by means of the at least one switch (102, 103) during rotational and/or upwardly and/or downwardly movement of the inner ring (8) and/or the printed circuit board (9) and/or the printed circuit board base (10) relative to the screw base (6) and/or the stop member (11).

8. Dispensing device (3) according to one of the preceding claims, **characterized in, that** the dispensing device (3) comprises at least one means for electronically and/or mechanically identifying the kind of reservoir (2) attached to the dispensing device (3), wherein a means for electronically identifying the kind of reservoir preferably comprises a barcode reader.

9. Dispensing device (3) according to one of the preceding claims, **characterized in, that** the dispensing device (3) comprises an antenna (14), to which especially the printed circuit board (9) and/or the electronics (95, 95a) is/are connected to.

10. Dispensing device (3) according to one of the preceding claims, **characterized in, that** it is provided with at least one means for at least storing information relating to the reservoir (2) and/or the dispense event of a substance or a pill (35) and/or a temperature and/or a humidity in the reservoir (2) and/or for at least transmitting information thereabout to an external device.

11. Dispensing device (3) according to one of the preceding claims, **characterized in, that** the dispensing device (3) comprises means allowing the device (3) to communicate via Global System for Mobile Communications (GSM).

12. Dispensing device (3) according to one of the preceding claims, **characterized in, that** it is a medical dispensing device and that it comprises means for communicating with a second medical device to coordinate the administration of medication from the dispensing device (3) and the second dispensing device and/or to receive information from the second device.

13. Dispensing device (3) according to one of the preceding claims, **characterized in, that** it comprises an electronic circuit (2500) provided with a transceiver (2510) that wirelessly transmits the information associated with the dispensing event to an external device.

14. Dispensing device (3) according to one of the preceding claims, **characterized in, that** it is part of a system comprising an external data base (2808), which stores information received from and to be submitted to the dispensing device (3) and to which the dispensing device (3) is, preferably wirelessly, communicating and vice versa.

15. Dispensing device (3) according to one of the preceding claims, **characterized in, that** it tracks at least one of a patient's medicine compliance data.
